# EUROPÄISCHE PATENTSCHRIFT

(11) **EP 1 648 603 B1**
(45) Veröffentlichungstag und Bekanntmachung des Hinweises auf die Patenterteilung: **11.06.2014**
(21) Anmeldenummer: 04763393.8
(22) Anmeldetag: 22.07.2004
(51) Int. Cl.: A61L 15/60, A61L 15/08

(54) **PULVERFÖRMIGE, WASSERABSORBIERENDE POLYMERE MIT MITTELS THERMOPLASTISCHEN KLEBSTOFFEN GEBUNDENEN FEINTEILCHEN**
POWDERY, WATER-ABSORBENT POLYMERS COMPRISING FINE PARTICLES BONDED BY MEANS OF THERMOPLASTIC ADHESIVES
POLYMERES PULVERULENTS HYDROPHILES, A FINES PARTICULES LIEES PAR DES ADHESIFS THERMOPLASTIQUES

(30) Priorität: 25.07.2003 DE 10334286
(43) Veröffentlichungstag der Anmeldung: 26.04.2006
(73) Patentinhaber: Evonik Degussa GmbH, 45128 Essen (DE)
(72) Erfinder: LOEKER, Frank, 47798 Krefeld (DE); BREMUS, Heinz, 47809 Krefeld (DE); SMITH, Scott, Greensboro, NC 27407 (US)
(74) Vertreter: Lang, Arne
(86) Internationale Anmeldenummer: PCT/EP2004/008184
(87) Internationale Veröffentlichungsnummer: WO 2005/011860

(56) Entgegenhaltungen:
- EP-A- 0 388 120
- WO-A-00/10619
- WO-A-01/74913
- WO-A-91/18042
- DE-A- 3 503 458
- DE-A- 10 026 861
- DE-A- 19 939 662
- DE-C1- 4 020 780
- US-A- 5 002 986

## Beschreibung

Die vorliegende Erfindung betrifft pulverförmige wasserabsorbierende Polymere, vorzugsweise Superabsorber, ein Verfahren zur Herstellung pulverförmiger wasserabsorbierender Polymere, die durch dieses Verfahren erhältlichen pulverförmigen wasserabsorbierenden Polymere, ein Transportverfahren, einen Verbund, chemische Produkte sowie die Verwendung eines thermoplastischen Klebstoffes.

Superabsorber sind wasserunlösliche, vernetzte Polymere, die in der Lage sind, unter Quellung und Ausbildung von Hydrogelen große Mengen an Wasser, wässrigen Flüssigkeiten, insbesondere Körperflüssigkeiten, vorzugsweise Urin oder Blut, aufzunehmen und unter Druck zurückzuhalten. Superabsorber absorbieren vorzugsweise mindestens das 100-fache ihres Eigengewichts an Wasser. Weitere Einzelheiten zu Superabsorbern sind in "Modern Superabsorbent Polymer Technology", F. L. Buchholz, A. T. Graham, Wiley-VCH, 1998" offenbart. Durch diese charakteristischen Eigenschaften sind diese wasserabsorbierenden Polymere hauptsächlich in Sanitärartikeln wie beispielsweise Babywindeln, Inkontinenzprodukten oder Damenbinden eingearbeitet.

Bei den gegenwärtig kommerziell verfügbaren Superabsorbern handelt es sich im wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind. Diese sind dadurch erhältlich, dass monomere Acrylsäure bzw. deren Salze in Gegenwart geeigneter Vernetzungsmittel radikalisch polymerisiert werden. Dabei können unterschiedliche Polymerisationsverfahren zur Anwendung gelangen, wie beispielsweise die Lösungspolymerisation, die Emulsionspolymerisation oder die Suspensionspolymerisation. Letztendlich werden durch diese unterschiedlichen Verfahren wasserabsorbierende Polymere in partikulärer Form mit einem Partikeldurchmesser in einem Bereich von 150 bis 850 µm erhalten, die dann in die Sanitärartikel eingearbeitet werden.

Um die Saug- und Quellfähigkeit dieser wasserabsorbierenden Polymerteilchen zu verbessern, wurden zahlreiche Verfahren beschrieben, in denen die Oberfläche der Polymerteilchen modifiziert wird. So ist beispielsweise aus DE-A-40 20 780 bekannt, die wasserabsorbierenden Polymerteilchen mit Alkylencarbonaten, die mit den Carboxylgruppen der Polymerteilchen reagieren können, umzusetzen. Die auf diese Weise bewirkte Oberflächennachvernetzung führt zu einer Erhöhung der Absorption der Polymere unter der Einwirkung eines Druckes.

Neben der Umsetzung der Polymerpartikel mit reaktiven Verbindungen werden im Stand der Technik auch zahlreiche Verfahren beschrieben, mit denen die Eigenschaften der wasserabsorbierenden Polymerteilchen durch eine Beschichtung mit anorganischen oder organischen Feinteilchen erzielt wird.

So wird in DE-A-35 03 458 beschrieben, dass die Absorptionskapazität, die Absorptionsgeschwindigkeit sowie die Gelfestigkeit von Superabsorberpartikeln durch das Aufbringen von inerten anorganischen Pulvermaterialien, wie beispielsweise Siliziumdioxid, in Gegenwart von Nachvemetzungsmitteln verbessert werden kann.

Zur Verminderung der Hygroskopizität und somit zur Verminderung des Verbackens der Polymerteilchen schlägt EP-A-0 388 120 vor, die Polymerteilchen mit einem porösen Pulver aus hochreinem Siliziumdioxid zu beschichten, wobei das Pulver eine mittlere Teilchengröße von 0,1 bis 30 µm und eine spezifische Oberfläche von 500 m²/g aufweist.

DE-A-199 39 662 lehrt, dass wasserabsorbierende Polymergebilde mit partikulären Cyclodextrinen und Zeolithen zu beschichten, um auf die Weise Verbindungen aus Körperflüssigkeiten, die durch einen unangenehmen Geruch gekennzeichnet sind, zu binden.

US5002886 offenbart Flüssigkeit absorbierende Polymerpartikeln. Zur Erhöhung der Absorptionsrate können diese Partikeln mit einer inerten, anorganischen, nicht-hydrophoben, Wasser unlöslichen Verbindung einer Partikelgröße kleiner als 150 Mikrometer nach Vernetzung und Agglomeration in einer Menge von 0,5 bis 20 Gew.% vermischt werden.

WO01/74913 offenbart pulverförmige, wasserabsorbierende Polymere. Die Polymerpulver kann Feinteilchen in einer Menge bis zu 15 Gew.% und einer mittleren Korngröße von kleiner 150 Mikrometer aufweisen.

All diesen Verfahren zur nachträglichen Modifizierung ist jedoch gemein, dass sie zu einer Freisetzung von Feinteilchen in Form von Staub führen, wobei diese Feinteilchen durch mechanische Belastung, wie zum Beispiel durch pneumatische Förderung und dadurch bedingten Abrieb der wasserabsorbierenden Polymerteilchen entstehen oder auf ein mangelndes Anhaften der anorganischen oder organischen Feinteilchen, die zur nachträglichen Modifizierung der Polymerteilchen aufgebracht wurden, an der Oberfläche der absorbierenden Feinteilchen resultieren.

Diese Freisetzung von Staub, insbesondere von Staub mit einer Korngröße von weniger als 10 µm ist aus inhalationstoxischen Gründen unerwünscht, Feinteilchen mit einer Partikelgröße von weniger als 100 µm verursachen das visuell sichtbare Stauben mit all seinen Folgeerscheinungen und führen zu Handlingproblemen im Produktions- und Verarbeitungsvertrieb. Vor allem die Fliessfähigkeit der mit anorganischen oder organischen Partikeln oberflächenmodifizierten Superabsorber wird durch die mangelhafte Anhaftung dieser Partikel an der Oberfläche der Superabsorberpartikel nachteilig beeinflusst.

Der vorliegenden Erfindung lag demnach die Aufgabe zugrunde, die sich aus dem Stand der Technik ergebenden Nachteile zu überwinden.

Insbesondere bestand die der vorliegenden Erfindung zugrunde liegende Aufgabe darin, ein Verfahren anzugeben, mit dem es möglich ist, mit anorganischen oder organischen Partikeln modifizierte Superabsorberpartikel herzustellen, die eine ausreichende Haftung der organischen oder anorganischen Partikel auf der Oberfläche der Superabsorberpartikel aufweisen.

Weiterhin bestand die Aufgabe der vorliegenden Erfindung darin, wasserabsorbierende Partikel bereitzustellen, die hervorragende Saugeigenschaften, beispielsweise hinsichtlich der Absorptionskapazität, der Permeabilität oder der Absorptionsgeschwindigkeit aufweisen und die bei einem Transport in Förderanlagen zur Herstellung von Hygieneartikel ein zufrieden stellendes Fließverhalten zeigen und nicht zur Staubbildung neigen.

Zudem bestand eine erfindungsgemäße Aufgabe darin, wasserabsorbierende Polymerteilchen bereitzustellen, die hervorragende Saugeigenschaften, beispielsweise hinsichtlich der Absorptionskapazität, der Permeabilität oder der Absorptionsgeschwindigkeit aufweisen und dazu durch eine gute mechanische Stabilität gekennzeichnet sind.

Außerdem sollen die wasserabsorbierenden Polymerteilchen bei ihrer Einarbeitung in einen Verbund umfassend eine Matrix aus einem faserförmigen Material eine ausreichende Fixierung innerhalb des Verbundes durch ein Anhaften der wasserabsorbierenden Polymerteilchen an den Fasern der Matrix ermöglichen. Auch war es eine Aufgabe der vorliegenden Erfindung, einen Verbund umfassend wasserabsorbierende Polymerteilchen bereitzustellen, der durch die hervorragenden Absorptionseigenschaften der wasserabsorbierenden Polymerteilchen eine im Vergleich zu den aus dem Stand der Technik bekannten Absorptionspartikeln mindestens vergleichbare Wasseraufnahme zeigt oder in dem eine zufrieden stellende Fixierung der wasserabsorbierenden Polymerteilchen innerhalb des Verbundes gewährleistet ist, wobei die in dem Verbund eingearbeiteten wasserabsorbierenden Polymerteilchen eine verbesserte Stabilität und/oder eine verringerte Staubneigung zeigen.

Eine der vorliegenden Erfindung zugrunde liegende Aufgabe bestand des Weiteren darin, chemische Produkte beinhaltend die wasserabsorbierenden Polymerteilchen bereitzustellen, die hervorragende Absorptionseigenschaften aufweisen und durch eine äußerst geringe Freisetzung von Staub, beispielsweise bei einer mechanischen Belastung, gekennzeichnet sind.

Außerdem bestand eine Aufgabe der vorliegenden Erfindung darin, ein wasserabsorbierendes Polymerteilchen bereit zu stellen, dass möglichst wenig durch die bei Transportprozessen im Rahmen der Herstellung dieser oder bei der Weiterverarbeitung in Dosiersystemen bei Airlaid- oder Windelmaschinen auftretenden mechanischen Beanspruchung beschädigt wird und Stäube bildet.

Die vorstehenden Aufgaben werden gelöst durch pulverförmige wasserabsorbierende Polymere, durch ein Verfahren zur Herstellung pulverförmiger wasserabsorbierender Polymere, durch die durch dieses Verfahren erhältlichen pulverförmigen wasserabsorbierenden Polymere, durch ein Transportverfahren, durch einen Verbund, durch chemische Produkte sowie durch die Verwendung eines thermoplastischen Klebstoffes nach den jeweiligen kategoriebildenden Ansprüchen. Vorteilhafte Ausführungsformen der erfindungsgemäßen pulverförmigen wasserabsorbierenden Polymere oder des erfindungsgemäßen Verfahrens zur Herstellung dieser pulverförmigen wasserabsorbierenden Polymere sind Gegenstand der abhängigen Ansprüche, die jeweils einzeln angewandt oder beliebig miteinander kombiniert werden können.

Die erfindungsgemäßen pulverförmigen wasserabsorbierenden Polymere beinhalten als Bestandteile:
- 0,01 bis 20 Gew.-%, vorzugsweise, 0,05 bis 15 Gew.-% und darüber hinaus bevorzugt 0,1 bis 10 Gew.-%, eines Feinteilchens mit einer durch Siebanalyse bestimmten Teilchengröße von weniger als 200 µm, vorzugsweise von weniger als 100 µm und besonders bevorzugt von weniger als 50 µm;
- 0,001 bis 10 Gew.-%, vorzugsweise 0,01 bis 5 Gew.-% und besonders bevorzugt 0,1 bis 1 Gew.-% eines thermoplastischen Klebstoffs,
- 60 bis 99,998 Gew.-%, vorzugsweise 70 bis 99 Gew.-% und besonders bevorzugt 80 bis 95 Gew.-% eines wasserabsorbierenden Polymerteilchens mit einer durch Siebanalyse Teilchengröße von mindestens 200 µm, vorzugsweise von mindestens 250 µm und besonders bevorzugt von mindestens 300 µm, wobei die Summe der vorstehenden Komponenten 100 Gew.-% beträgt,
wobei
die Feinteilchen mit der Oberfläche des wasserabsorbierenden Polymerteilchens über den thermoplastischen Klebstoff verbunden sind und die pulverförmigen wasserabsorbierenden Polymere entweder
einen gemäß der hierin beschriebenen Testmethode bestimmten Fließ-Wert (FFC-Wert) im Bereich von 1 bis 13, vorzugsweise im Bereich von 3 bis 9 und besonders bevorzugt im Bereich von 4 bis 8 und darüber hinaus bevorzugt im Bereich von 5 bis 7, oder
einen gemäß der hierin beschriebenen Testmethode bestimmten Staubanteil von höchstens 6, vorzugsweise höchstens 4, besonders bevorzugt höchstens 2, zeigen. Vorzugsweise zeigen die pulverförmigen wasserabsorbierenden Polymerteilchen sowohl Fließwert (FFC-Wert) und Staubanteil in den vorgenannten Bereichen. Gemäß einer Ausführungsform der vorliegenden Erfindung handelt es sich bei diesen Feinteilchen um so genannte Superabsorberfeinteilchen, die bei Transport-oder Siebschritten im Rahmen der Herstellung und Konfektionierung von Superabsorbern anfallen.

In einer weiteren Ausführung der vorliegenden Erfindung ist es bevorzugt, dass die Feinteilchen nicht Superabsorber sind und insbesondere nicht auf wasserabsorbierenden, vernetzten, teilneutralisierten Polyacrylsäuren oder deren Salzen basieren. So werden beispielsweise zum Geruchsmanagement von Hygieneartikeln Cyclodextrine als Feinteilchen bei der Konfektionierung eingesetzt, wie in EP 0 691 856 Bl beschrieben.

Bei den vorzugsweise nicht auf wasserabsorbierenden, vernetzten, teilneutralisierten Polyacrylsäuren oder deren Salzen basierenden Feinteilchen handelt es sich vorzugsweise um organische, von wasserabsorbierenden Polymeren verschiedene, oder anorganische Feinteilchen.

Als organisches Material kann jedes dem Fachmann bekannte, partikuläre organische Material in den erfindungsgemäßen pulverförmigen wasserabsorbierenden Polymeren enthalten sein, welches üblicherweise zur Modifizierung der Eigenschaften wasserabsorbierender Polymere eingesetzt wird. Zu den bevorzugten organischen Feinteilchen gehören Cyclodextrine oder deren Derivate sowie Polysaccharide. Außerdem sind Cellulose und Cellulosederivate wie CMC, Celluloseether bevorzugt.

Als Cyclodextrine oder Cyclodextrinderivate sind dabei diejenigen Verbindungen bevorzugt, die in DE-A-198 25 486 auf der Seite 3, Zeile 51 bis Seite 4, Zeile 61 offenbart sind. Der vorstehend genannte Abschnitt dieser veröffentlichten Patentanmeldung wird hiermit als Referenz eingeführt und gilt als Teil der Offenbarung der vorliegenden Erfindung. Besonders bevorzugte Cyclodextrine sind nicht derivatisierte α-, β-, γ- oder δ-Cyclodextrine. Bei der Verwendung organischer Feinteilchen ist es besonders bevorzugt, dass diese Feinteilchen in einer Menge in einem Bereich von 0,1 bis 1 Gew.-%, darüber hinaus bevorzugt in einer Menge in einem Bereich 0,25 bis 0,75 Gew.-% und darüber hinaus noch mehr bevorzugt in einem Bereich von 0,4 bis 0,6 Gew.-% in den pulverförmigen wasserabsorbierenden Polymeren enthalten sind. Besonders haben sich die in den erfindungsgemäßen Beispielen angegebenen Mengenverhältnisse als vorteilhaft erwiesen.

Als anorganisches Material kann jedes dem Fachmann bekannte, partikuläre anorganische Material in den erfindungsgemäßen pulverförmigen wasserabsorbierenden Polymeren enthalten sein, welches üblicherweise zur Modifizierung der Eigenschaften wasserabsorbierender Polymere eingesetzt wird. Zu den bevorzugten anorganischen Feinteilchen gehören Silikate, insbesondere Gerüstsilikate wie Zeolithe oder Silikate, die durch Trocknung wässriger Kieselsäurelösungen oder Kieselsolen erhalten wurden, beispielsweise die kommerziell erhältlichen Produkte wie Fällungskieselsäuren und pyrogene Kieselsäuren, beispielsweise Aerosile mit einer Teilchengröße im Bereich von 5 bis 50 nm, vorzugsweise im Bereich von 8 bis 20 nm wie "Aerosil 200" der Degussa AG, Aluminate, Titandioxide, Zinkoxide, Tonmaterialien und weitere dem Fachmann geläufige Mineralien sowie kohlenstoffhaltige anorganische Materialien.

Bevorzugte Silikate sind alle natürlichen oder synthetischen Silikate, die in "Holleman und Wiberg, Lehrbuch der Anorganischen Chemie, Walter de Gruyter-Verlag, 91.-100. Auflage, 1985" auf den Seiten 750 bis 783, als Silicate offenbart sind. Der vorstehend genannte Abschnitt dieses Lehrbuchs wird hiermit als Referenz eingeführt und gilt als Teil der Offenbarung der vorliegenden Erfindung.

Besonders bevorzugte Silikate sind die Zeolithe. Als Zeolithe können alle dem Fachmann bekannten synthetischen oder natürlichen Zeolithe eingesetzt werden. Bevorzugte natürliche Zeolithe sind Zeolithe aus der Natrolith-Gruppe, der Harmoton-Gruppe, der Mordenit-Gruppe, der Chabasit-Gruppe, der Faujasit-Gruppe-(Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS.

Als Zeolithe können Zeolithe des so genannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie ß-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Als Aluminate werden vorzugsweise die in der Natur vorkommenden Spinelle, insbesondere gewöhnlicher Spinell, Zinkspinell, Eisenspinell oder Chromspinell eingesetzt.

Bevorzugte Titandioxide ist das reine Titandioxid in den Kristallformen Rutil, Anatas und Brookit, sowie eisenhaltige Titandioxide wie beispielsweise Ilmenit, calciumhaltige Titandioxide wie Titanit oder Perowskit.

Bevorzugte Tonmaterialien sind diejenigen, die in "Holleman und Wiberg, Lehrbuch der Anorganischen Chemie, Walter de Gruyter-Verlag, 91.-100. Auflage, 1985" auf den Seiten 783 bis 785, als Tonmaterialien offenbart sind. Der vorstehend genannte Abschnitt dieses Lehrbuchs wird hiermit als Referenz eingeführt und gilt als Teil der Offenbarung der vorliegenden Erfindung. Besonders bevorzugte Tonmaterialien sind Kaolinit, Illit, Halloysit, Montmorillonit sowie Talk.

Weiterhin sind als anorganische Feinteilchen die Metallsalze der Mono-, Oligo-und Polyphosphorsäuren erfindungsgemäß bevorzugt. Hierunter sind insbesondere die Hydrate bevorzugt, wobei die Mono- bis Deca-Hydrate und Tri-Hydrate besonders bevorzugt sind. Als Metalle kommen insbesondere Alkali- und Erdalkalimetalle in Betracht, wobei die Erdalkalimetalle bevorzugt sind. Hierunter sind Mg und Ca bevorzugt und Mg besonders bevorzugt. Im Zusammenhang mit Phosphaten, Phosphorsäuren und deren Metallverbindungen wird auf "Holleman und Wiberg, Lehrbuch der Anorganischen Chemie, Walter de Gruyter-Verlag, 91.-100. Auflage, 1985" auf den Seiten 651 bis 669 verwiesen. Der vorstehend genannte Abschnitt dieses Lehrbuchs wird hiermit als Referenz eingeführt und gilt als Teil der Offenbarung der vorliegenden Erfindung.

Bevorzugte kohlenstoffhaltige, jedoch nicht organische Materialien sind diejenigen reinen Kohlenstoffe, die in "Holleman und Wiberg, Lehrbuch der Anorganischen Chemie, Walter de Gruyter-Verlag, 91.-100. Auflage, 1985" auf den Seiten 705 bis 708 als Graphite genannt sind. Der vorstehend genannte Abschnitt dieses Lehrbuchs wird hiermit als Referenz eingeführt und gilt als Teil der Offenbarung der vorliegenden Erfindung. Besonders bevorzugte Graphite sind künstliche Graphite wie beispielsweise Koks, Pyrographit, Aktivkohle oder Ruß.

Die in den erfindungemäßen pulverförmigen wasserabsorbierenden Polymere enthaltenen Feinteilchen können in Form von Fasern, Schäumen oder Teilchen vorliegen, wobei Fasern und Teilchen bevorzugt und Teilchen besonders bevorzugt sind. Werden als Feinteilchen organische Polysaccharide, wie beispielsweise Cellulose-Feinteilchen, eingesetzt, so ist es bevorzugt, dass die Feinteilchen in Form von Fasern eingesetzt werden. Diese Fasern weisen vorzugsweise einen Titer im Bereich von 0,5 bis 6 und besonders bevorzugt von 0,7 bis 4 detex auf. Weitere Einzelheiten zu geeigneten Fasern sind in EP-A-0 273 141 offenbart.

Bei der Verwendung organischer oder anorganischer Feinteilchen oder deren nachfolgend beschriebenen Mischungen ist es besonders bevorzugt, dass diese Feinteilchen in einer Menge in einem Bereich von 0,1 bis 1 Gew.-%, darüber hinaus bevorzugt in einer Menge in einem Bereich von 0,25 bis 0,75 Gew.-% und darüber hinaus noch mehr bevorzugt in einem Bereich von 0,4 bis 0,6 Gew.-% in den pulverförmigen wasserabsorbierenden Polymeren enthalten sind. Besonders haben sich die in den erfindungsgemäßen Beispielen angegebenen Mengenverhältnisse, insbesondere für die vorstehend aufgeführten organischen und anorganischen Einzelverbindungen als vorteilhaft erwiesen.

In einer bevorzugten Ausführungsform der erfindungsgemäßen pulverförmigen, wasserabsorbierenden Polymere beinhalten diese als Bestandteil eine Mischung aus einem organischen und einem anorganischen Feinteilchen.

In einer bevorzugten Ausführungsform der erfindungsgemäßen pulverförmigen wasserabsorbierenden Polymer beinhaltet das vorzugsweise nicht auf wasserabsorbierenden, vernetzten Poly(meth)acrylaten basierende Feinteilchen zu mindestens 80 Gew.-%, vorzugsweise zu mindestens 90 Gew.-% und darüber hinaus bevorzugt zu mindestens 95 Gew.-%, jeweils bezogen auf das Gewicht des vorzugsweise nicht auf wasserabsorbierenden, vernetzten Poly(meth)acrylaten basierenden Feinteilchens, ein anorganisches Feinteilchen.

In einer anderen bevorzugten Ausführungsform der erfindungsgemäßen pulverförmigen wasserabsorbierenden Polymere beinhaltet das vorzugsweise nicht auf wasserabsorbierenden, vernetzten Poly(meth)acrylaten basierende Feinteilchen zu mindestens 80 Gew.-%, vorzugsweise zu mindestens 90 Gew.-% und darüber hinaus bevorzugt zu mindestens 95 Gew.-%, jeweils bezogen auf das Gewicht des vorzugsweise nicht auf wasserabsorbierenden, vernetzten Poly(meth)acrylaten basierenden Feinteilchens, ein organisches Feinteilchen.

Es ist erfindungsgemäß weiterhin bevorzugt, dass die vorzugsweise nicht auf wasserabsorbierenden, vernetzten, teilneutralisierte Polyacrylsäuren oder deren Salzen basierenden Feinteilchen eine nach der BET-Methode bestimmte spezifische Oberfläche in einem Bereich von 30 bis 850 m²/g, vorzugsweise in einem Bereich von 40 bis 500 m²/g, besonders bevorzugt in einem Bereich von 100 bis 300 m²/g und darüber hinaus bevorzugt in einem Bereich von 150 bis 250 m²/g aufweisen. Im allgemeinen und in dem Fall, dass es sich bei den Feinteilchen um Sipernate oder Aerosile handelt liegt die Oberfläche in einem Bereich von 30 bis 850 m²/g, vorzugsweise in einem Bereich von 40 bis 500 m²/g, besonders bevorzugt in einem Bereich von 100 bis 300 m²/g und wird mit Stickstoff in einem Areameter nach ISO 5794, Annex D bestimmt.

In einer bevorzugten Ausführungsform der erfindungsgemäßen pulverförmigen wasserabsorbierenden Polymere weisen mindestens 90 Gew.-%, vorzugsweise mindestens 95 Gew.-% und darüber hinaus bevorzugt mindestens 99 Gew.-% der vorzugsweise nicht auf wasserabsorbierenden, vernetzten Polyacrylaten basierenden Feinteilchen eine Partikelgröße von weniger als 200 µm, besonders bevorzugt von weniger als 100 µm und darüber hinaus bevorzugt von weniger als 1 µm und darüber hinaus bevorzugt von weniger als 500 nm und darüber hinaus noch mehr bevorzugt von weniger als 100 nm auf. Die Sipernate weisen eine Teilchengröße im Bereich von 10 bis 180 µm, vorzugsweise im Bereich von 20 bis 150 µm und besonders bevorzugt im Bereich von 30 bis 110 µm auf. Die Teilchengröße lässt sich nach ASTM C 690-1992 mit einem Multisizer bestimmen. In einer anderen Ausgestaltung der vorliegenden Erfindung weisen die Sipernate eine nach ASTM C 690-1992 bestimmte mittlere Teilchengröße im Bereich von 1 bis 40 µm, vorzugsweise im Bereich von 2 bis 30 µm, besonders bevorzugt im Bereich von 3 bis 20 µm auf.

Unter einem "thermoplastischen Klebstoff" wird erfindungsgemäß vorzugsweise ein Material verstanden, das unterhalb einer bestimmten Temperatur keine klebrigen Eigenschaften aufweist. Oberhalb einer bestimmten Temperatur, vorzugsweise oberhalb der Schmelztemperatur des Materials, entfaltet das Material jedoch klebrige Eigenschaften oder Hafteigenschaften, wobei diese Eigenschaften nach der Verbindungen zweier Substrate im geschmolzenen Zustand auch im erstarrten Zustand erhalten bleiben. Vorzugsweise treten die Klebe- bzw. Hafteigenschaften mindestens 10°C, vorzugsweise mindestens 20° und besonders bevorzugt in einem Bereich von 21 bis 100°C über dem durch Differentialcalometrie (DSC) bestimmten Schmelzpunkt des als thermoplastischen Klebstoffs eingesetzten Materials oder Materialmischung auf. Es ist im Rahmen der vorliegenden Erfindung ebenfalls möglich, thermoplastische Klebstoffe einzusetzen, die aus zwei oder mehr unterschiedlichen Verbindungen bestehen. Die zwei oder mehr Verbindungen können sich dabei im chemischen Aufbau oder im Molekulargewicht oder in beidem unterscheiden.

Die thermoplastischen Klebstoffe werden vorzugsweise als Teilchen eingesetzt. Von diesen Teilchen besitzen vorzugsweise mindestens 50 Gew.-% und besonders bevorzugt mindestens 70 Gew.-% der Teilchen eine Teilchengröße im Bereich von 10 bis 500 µm, vorzugsweise 25 bis 250 µm und darüber hinaus bevorzugt 40 bis 120 µm. Die Teilchengröße kann bis zu einer Größe von 20 µm durch Siebanalyse bestimmt werden. Bei Teilchengrößen von weniger als 20 µm kann Lichtstreuung zur Bestimmung der Teilchengröße verwendet werden.

Als thermoplastischen Klebstoffe eignen sich in der Regel polymere Materialien mit einem Molekulargewicht von mehr als etwa 1.000 g/Mol, die eine entsprechende Schmelztemperatur aufweisen und bei einer entsprechenden Anwendungstemperatur keine Zersetzung oder anderweitige für die klebende Wirkung nachteilige Veränderung des Molekülaufbaus zeigen.

Das durch Gelpermeationschromatographie (GPC) bestimmte Zahlenmittel des Molekulargewichts (Mₙ) der als thermoplastischen Klebstoffe einsetzbaren Polymeren liegt vorzugsweise zwischen etwa 10.000 und etwa 1.000.000, besonders bevorzugt zwischen etwa 20.000 und etwa 300.000 und darüber hinaus bevorzugt zwischen etwa 50.000 und etwa 150.000 g/Mol.

Die Molekulargewichtsverteilung der genannten Polymeren, wie sie ebenfalls durch Gelpenneationschromatographie (GPC) ermittelt werden kann, kann monomodal sein. Gegebenenfalls kann ein als thermoplastischer Klebstoff einsetzbares Polymeres auch eine bi- oder höhermodale Verteilung aufweisen.

Die im Rahmen der vorliegenden Erfindung als thermoplastischen Klebstoffe einsetzbaren Verbindungen weisen zu einem Anteil von mindestens 60 Gew.-%, vorzugsweise zu einem Anteil von 80 Gew.-% und darüber hinaus bevorzugt zu einem Anteil von 100 Gew.-%, jeweils bezogen auf das Gesamtgewicht des thermoplastischen Klebstoffes, eine Schmelztemperatur nach ISO 11357 von mindestens 50°C, vorzugsweise von mindestens 60°C und darüber hinaus bevorzugt von mindestens 70°C auf, wobei vorzugsweise eine Schmelztemperatur nach ISO 11357 von 300°C, besonders bevorzugt von 250°C und darüber hinaus bevorzugt von 200°C nicht überschritten wird.

Die im Rahmen der vorliegenden Erfindung einsetzbaren thermoplastischen Klebstoffe sollen bei Temperaturen von bis zu etwa 30°C, vorzugsweise jedoch bei darüber liegenden Temperaturen, beispielsweise bei bis zu 40°C oder bis zu 50°C nicht nur fest, sondern auch eine nichtklebrige Oberfläche aufweisen.

Schon wenig oberhalb der Schmelztemperatur sollte der thermoplastische Klebstoff schon relativ niedrigviskos sein. Vorzugsweise sollte seine Schmelzviskosität nach Brookfield (ASTM E 28) mit einer 27ger Spindel bei einer Temperatur von 160°C, kleiner als 2000 Pas, vorzugsweise kleiner als 1200 Pas und besonders bevorzugt kleiner als 600 Pas sein.

Als thermoplastischer Klebstoff wird im Rahmen der vorliegenden Erfindung vorzugsweise ein Material eingesetzt, das zu mindestens 10 Gew.-%, vorzugsweise zu mindestens 50 Gew.-% und besonders bevorzugt zu mindestens 90 Gew.-%, jeweils bezogen auf das Gesamtgewicht des thermoplastischen Klebstoffes, eines Polymeren ausgewählt aus Polyurethanen, Polyestern, Polyamiden, Polyolefinen, Polyvinylestern, Polyethern, Polystyrolen, Polyimiden, insbesondere Polyetherimide, Schwefelpolymeren, insbesondere Polysulfone, Polyacetale, insbesondere Polyoxymethylene, Fluorkunststoffe, insbesondere Polyvinylidenfluorid, Styrol-Olefin-Copolymeren, Polyacrylaten oder Ethylen-Vinylacetat-Copolymeren oder Gemischen aus zwei oder mehr der genannten Polymeren aufweist, wobei unter diesen Polymeren Polykondensate und unter diesen Polyester besonders bevorzugt sind.

Als Polyurethane sind im Rahmen der vorliegenden Verbindung alle Polymeren zu verstehen, die wenigstens zwei Urethangruppen im Polymerrückgrat aufweisen.

Als Polyurethane sind im Rahmen der vorliegenden Erfindung alle dem Fachmann auf dem Gebiet der Polyurethanchemie bekannten thermoplastischen Polyurethane geeignet, insbesondere solche Polyurethane, wie sie üblicherweise im Rahmen der Herstellung thermoplastischer Formkörper, insbesondere von Folien, oder zur thermoplastischen Beschichtung von Oberflächen eingesetzt werden. Geeignet sind beispielsweise Polyesterpolyurethane oder Polyetherpolyurethane, wie sie durch Umsetzung von Dicarbonsäuren mit entsprechenden polyfunktionellen Alkoholen, insbesondere difunktionellen Alkoholen, beispielsweise difunktionellen Polyethern wie Polyethylenoxid, zu Polyether- oder Polyesterpolyolen und anschließender Umsetzung der entsprechenden Polyether- oder Polyesterpolyole mit di- oder polyfunktionellen Isocyanaten erhältlich sind. Besonders bevorzugte thermoplastische Polyurethane sind diejenigen Polyurethane, die von der Firma Kern GmbH, Großmaischeid, Deutschland, unter den Produktbezeichnungen "TPU D", "TPU 93A", "TPU 80A" und "TPU GF20" vertrieben werden. Ein weiteres bevorzugtes thermoplastisches Polyurethan ist das von der Firma Schaetti AG, Zürich, Schweiz, unter der Produktbezeichnung "SchaettiFix®" vertrieben Polyurethan mit der Produktnummer "6005".

Als Polyester sind im Rahmen der vorliegenden Verbindung alle Polymeren zu verstehen, die wenigstens zwei Estergruppen und keine Urethangruppen im Polymerrückgrat aufweisen. Als Polyester sind im Rahmen der vorliegenden Erfindung alle dem Fachmann bekannten thermoplastischen Polyester geeignet, insbesondere solche Polyester, wie sie üblicherweise im Rahmen der Herstellung thermoplastischer Formkörper, insbesondere von Folien, oder zur thermoplastischen Beschichtung von Oberflächen eingesetzt werden. Geeignet sind beispielsweise Polyester, wie sie durch Umsetzung von Dicarbonsäuren mit entsprechenden polyfunktionellen Alkoholen, insbesondere difunktionellen Alkoholen, beispielsweise difunktionellen Polyethern wie Polyethylenoxid erhältlich sind. Hierunter sind Polyester bevorzugt, die auf Terephthalsäure oder eines Derivats davon, Isophthalsäure oder eines Derivats davon, Adipinsäure oder Isophthalsäure oder eines Derivats davon, Adipinsäure oder eines Derivats davon und mindestens einem Polyol, vorzugsweise Butandiol oder Ethylenglykol oder deren Mischung, bestehen. In einer Ausführung des Esters ist es bevorzugt, dass dieser das Terephthalsäure oder ein Derivats davon, Isophthalsäure oder ein Derivats davon oder deren Mischungen in einer Menge im Bereich von 30 bis 80 Gew.-% und vorzugsweise in einer Menge im Bereich von 40 bis 65 Gew.-% beinhaltet sind. Eine weitere Ausführungsform des Esters beinhaltet dieser Adipinsäure oder eine Derivats davon in einer Menge im Bereich von 3 bis 20 Gew.-% und bevorzugt in einer Menge im Bereich von 5 bis 15 Gew.-%. Weiterhin beinhaltet eine andere Ausführungsformdes Esters mindestens ein Polyol, vorzugsweise Butandiol oder Ethylenglykol oder deren Mischung, in einer Menge im Bereich von 25 bis 55 Gew.-% und bevorzugt in Menge im Bereich von 30 bis 40 Gew.-%. Die vorstehenden Gew.-%-Angaben beziehen sich immer auf den jeweiligen Ester.

Besonders bevorzugte thermoplastische Polyester sind diejenigen Polyester, die von der Firma Kern GmbH, Großmaischeid, Deutschland, unter den Produktbezeichnungen "PET", "PBT", "PBT V0", "PC", "PC V0", "PC FDA", "PC GF10 V0" und "PC GF30" vertrieben werden. Besonders bevorzugt sind weiterhin diejenigen thermoplastischen Polyester, die von der Firma Schaetti AG, Zürich, Schweiz, unter der Produktbezeichnung "SchaettiFix" unter den Produktnummern "373", "374", "376", "386", "386F", und "399" vertrieben werden. Auch bevorzugt sind die thermoplastischen Copolyester, die von der Firma Degussa AG, Düsseldorf, Deutschland unter den Produktbezeichnungen "Dynacoll 7000", "Dynacoll 73 80", "Dynacoll 7340", "Vestamelt 4280", "Vestamelt 4481", "Vestamelt 4580" und "Vestamelt 4680/4681" vertrieben werden.

Als Polyamide sind im Rahmen der vorliegenden Erfindung alle thermoplastischen Polyamide geeignet, wie sie durch Umsetzung geeigneter Di- oder Polycarbonsäuren mit entsprechenden Aminen erhältlich sind. Im Rahmen einer bevorzugten Ausführungsform der vorliegenden Erfindung werden Polyamiden eingesetzt, die zumindest anteilig unter Verwendung von Dimerfettsäuren als Dicarbonsäuren hergestellt wurden. Im Rahmen einer weiteren bevorzugten Ausführungsform der vorliegenden Erfindung werden als Schmelzklebstoffe Nylontypen eingesetzt, beispielsweise Nylon-6,6, Nylon-6,9 oder Nylon-6,12.

Besonders bevorzugte thermoplastische Polyamide sind diejenigen Polyamide, die von der Firma Kern GmbH, Großmaischeid, Deutschland, unter den Produktbezeichnungen "PA 6 E", "PA 6", "PA 6 flex", "PA 6 V0", "PA 6 MoS2", "PA 6 M30", "PA 6 M30 VO", "PA 6 G 210", PA 6 G 210H", "PA 6 G 212", "PA 6 G 216", "PA 6 G 210 Öl", "PA 6 G 210 AX", PA 6 G 210 MoS2", "PA 6 G 210 AX", "PA 66 E", "PA 66", "PA 66 H", "PA 66 PE", "PA 6/6T", "PA 12 E", "PA 12" und "PA 12 G" vertrieben werden. Besonders bevorzugt sind weiterhin diejenigen thermoplastischen Polyamide, die von der Firma Schaetti AG, Zürich, Schweiz, unter der Produktbezeichnung "SchaettiFix®" und den Produktnummern "5018", "5047", "5045", "5005", "5000", "5010", und "5065" vertrieben werden. Weitere bevorzugte Polyamide sind die thermoplastischen Polyamide, die von der Firma Degussa AG, Düsseldorf, Deutschland unter den Produktbezeichnungen "Vestamelt 171", "Vestamelt 250", "Vestamelt 251", "Vestamelt 253", "Vestamelt 350", "Vestamelt 351", "Vestamelt 353", "Vestamelt 430", "Vestamelt 432", "Vestamelt 450", "Vestamelt 451", "Vestamelt 470", "Vestamelt 471", "Vestamelt 640", "Vestamelt 722", "Vestamelt 730", "Vestamelt 732", "Vestamelt 733", "Vestamelt 742", "Vestamelt 750", "Vestamelt 753", "Vestamelt 755", "Vestamelt 840", "Vestamelt X1301 ", "Vestamelt 3041", "Vestamelt 3261", "Vestamelt X4685", "Vestamelt X7079" und "Vestamelt X1010" vertrieben werden.

Die im Rahmen der vorliegenden Erfindung geeigneten Polyolefine sind beispielsweise durch radikalische oder koordinative Polymerisation von α-Olefinen, insbesondere von Ethylen oder Propylen, erhältlich. Im Rahmen der vorliegenden Erfindung eignen sich dabei zum Einsatz als thermoplastischer Klebstoff sowohl Homopolymere als auch Copolymere. Wenn als thermoplastischer Klebstoff Copolymere eingesetzt werden sollen, so ist es im Rahmen der vorliegenden Erfindung bevorzugt, wenn derartige thermoplastischen Klebstoffe zumindest einen Anteil an ataktischen Copolymeren enthalten, vorzugsweise jedoch im wesentlichen aus ataktischen Copolymeren bestehen. Zur Herstellung entsprechender Verbindungen geeignete Verfahren sind dem Fachmann bekannt.

Besonders bevorzugte thermoplastische Polyolefine sind diejenigen Polyethylene und Polypropylene, die von der Finna Kern GmbH, Großmaischeid, Deutschland, unter den Produktbezeichnungen "PE UHMW", "PE HMW", PE HMW ELS", "PE HD", "PE LD", "PP-H", "PP-C", "PP-C HI", "PP V2", "PP M20" und "PP M40" vertrieben werden. Besonders bevorzugt sind weiterhin diejenigen thermoplastischen Polyethylene, die von der Firma Schaetti AG, Zürich, Schweiz, unter der Produktbezeichnung "SchaettiFix®" und den Produktnummer "1800", "1820", "1822", "1825", "120" und "140" vertrieben werden.

Als Polyvinylester sind im Rahmen der vorliegenden Erfindung insbesondere die Polymere und Copolymere des Vinylacetats geeignet. Im Rahmen einer bevorzugten Ausführungsform werden Copolymere von Vinylacetat mit α-Olefinen, insbesondere mit Ethylen eingesetzt. Vorzugsweise weisen die Copolymeren einen Gehalt an Vinylacetat von etwa 15 bis etwa 40%, insbesondere etwa 18 bis etwa 25% auf. Der Schmelzeindex derartiger Polymere liegt vorzugsweise bei etwa 150 bis etwa 500, gemessen nach ASTMD 1238.

Besonders bevorzugte thermoplastische Polyvinylester sind diejenigen Polyvinylester, die von der Firma Schaetti AG, Zürich, Schweiz, unter der Produktbezeichnung "SchaettiFix®" und den Produktnummern "2047", "2048", "1303", und "2050" vertrieben werden.

Im Rahmen der vorliegenden Erfindung geeignete Polyether sind beispielsweise Polyethylenoxid, Polypropylenoxid, Polybutylenoxid oder Polytetrahydrofuran, insbesondere mit einem Molekulargewicht von mehr als etwa 5.000.

Als Polystyrole sind beispielsweise die Polymeren von Styrol oder α-Methylstyrol geeignet.

Ebenfalls als Polymere für die thermoplastischen Klebstoff geeignet sind die Styrol-Olefin- Blockcopolymeren, wie sie durch Copolymerisation von Styrol mit Mono- oder Diolefinen, insbesondere Butadien, erhältlich sind. Besonders geeignet sind in diesem Zusammenhang die als Synthesekautschuk bezeichneten Polymeren, wie sie aus der Copolymerisation von Styrol und Butadien oder Styrol und Isopren erhältlich sind. Ebenfalls geeignet sind im Rahmen der vorliegenden Erfindung Synthesekautschuke der genannten Art, die einer teilweisen oder vollständigen Hydrierung unterzogen wurden. Im Rahmen der vorliegenden Erfindung als thermoplastische Klebstoffe einsetzbare Synthesekautschuk weisen vorzugsweise ein Zahlenmittel des Molekulargewichts von etwa 70.000 bis etwa 200.000, beispielsweise etwa 80.000 bis etwa 150.000 auf.

Besonders bevorzugte thermoplastische Polymere auf Basis von Styrol sind diejenigen Styrolpolymere, die von der Firma Kern GmbH, Großmaischeid, Deutschland, unter den Produktbezeichnungen "PS", "PS V2", "SB", "SB V0", "SB ELS", "ABS", "ABS V0", "ABS EMV", "SAN", "ASA" und "ASA HI" vertrieben werden.

Die Begriffe "Polyacrylat" oder "Polyacrylate", wie sie im Rahmen des vorliegenden Textes im Zusammenhang mit dem thermoplastischen Klebstoff benutzt werden, beziehen sich im folgenden sowohl auf Polymere oder Copolymere der Acrylsäure und/oder ihrer Derivate als auch auf Polymere oder Copolymere der Methacrylsäure und/oder ihrer Derivate.

Polyacrylate lassen sich herstellen, indem Acrylsäure und/oder Methacrylsäure und/oder Derivate von Acrylsäure und/oder Methacrylsäure, beispielsweise deren Ester mit mono- oder polyfunktionellen Alkoholen, jeweils alleine oder als Gemisch aus zwei oder mehr davon, auf bekannte Weise, beispielsweise radikalisch oder ionisch, polymerisiert werden.

Im Rahmen der vorliegenden Erfindung können als thermoplastische Klebstoffe auf Basis von Polyacrylaten Homopolymere oder Copolymere eingesetzt werden, die neben den Acrylsäureestern (Acrylaten) noch Styrol, Acrylnitril, Vinylacetat, Vinylpropionat, Vinylchlorid, Vinylidenchlorid, Ethylen, Propylen und/oder Butadien aufweisen.

Als Monomere kommen bei der Herstellung auf Polyacrylaten basierenden thermoplastischen Klebstoffe insbesondere Methacrylat, Ethylacrylat, n-Butylacrylat, Isobutylacrylat, tert.-Butylacrylat, Hexylacrylat, 2-Ethylhexylacrylat oder Laurylacrylat in Frage. Gegebenenfalls können als weitere Monomere noch Acrylsäure, Methacrylsäure, Acrylamid oder Methacrylamid in geringen Mengen bei der Polymerisation zugegeben werden.

Gegebenenfalls können noch weitere Acrylate und/oder Methacrylate mit einer oder mehreren funktionellen Gruppen bei der Polymerisation anwesend sein. Beispielsweise sind dies Maleinsäure, Itaconsäure, Butandioldiacrylat, Hexandioldi-acrylat, Triethylenglycoldiacrylat, Tetraethylenglycoldiacrylat, Neopentylglycoldiacrylat, Trimethylolpropantriacrylat, 2-Hydroxyethylacrylat, 2- Hydroxyethylmethacrylat, Hydroxypropylacrylat, Propylenglycolmethacrylat, Butandiolmonoacrylat, Ethyldiglycolacrylat sowie, als sulfonsäuregruppentragendes Monomeres, beispielsweise 2-Acrylamido-2-methylpropansulfonsäure. Besonders bevorzugt sind Acrylester-Vinylester- Copolymere, Acrylester-Styrol-Copolymere oder Acrylester-Methacrylester-Copolymere.

Im Rahmen einer weiteren bevorzugten Ausführungsform sind in den erfindungsgemäßen pulverförmigen, wasserabsorbierenden Polymeren als thermoplastische Klebstoffe auf der Basis von Polyacrylaten Copolymerisate von Acrylsäure und Styrol enthalten, wobei das Monomerenverhältnis in derartigen Schmelzklebern etwa 70 : 30 (Styrol: Acrylsäure) beträgt.

Die in den erfindungsgemäßen pulverförmigen, wasserabsorbierenden Polymeren enthaltenen thermoplastischen Klebstoffe beinhalten mindestens eine der oben genannten Verbindungen. Es ist im Rahmen der vorliegenden Erfindung jedoch ebenso gut möglich, dass die eingesetzten thermoplastischen Klebstoffe zwei oder mehr der genannten Verbindungen enthalten.

Im Rahmen einer weiteren bevorzugten Ausführungsform der Erfindung enthält der thermoplastische Klebstoff mindestens zwei Polymere ausgewählt aus der Gruppe bestehend aus Vinylacetat-Homopolymere, Vinylacetat-Copolymeren, Poly-α-Olefinen, Polyacrylaten, Polymethacrylaten, Polyacrylsäureestern, Poly-methacrylsäureestern, Polyethern, Polyestern, Polyamiden oder Polyurethanen.

Neben einem oder mehreren der oben genannten Polymeren kann ein thermoplastischer Klebstoff, wie er in den erfindungsgemäßen pulverförmigen, wasserabsorbierenden Polymeren enthalten ist, noch weitere Zusatzstoffe beinhalten, die eine Modifizierung der Klebeigenschaften erlauben. Hierzu sind beispielsweise die so genannten Täckifier-Harze geeignet, die sich in natürliche Harze und synthetische Harze (Kunstharze) unterteilen lassen. Geeignete Tackifier-Harze sind beispielsweise Alkydharze, Epoxidharze, Melaminharze, Phenolharze, Urethanharze, Kohlenwasserstoffharze sowie natürliche Harze aus Kolophonium, Holzterpentinöl und Tallöl. Als synthetische Kohlenwasserstoffharze sind beispielsweise Ketonharze, Cumaron-Indenharze, Isocyanatharze und Terpen-Phenolharze geeignet. Im Rahmen der vorliegenden Erfindung sind synthetische Harze bevorzugt.

Derartige Harze können in den in den erfindungsgemäßen pulverförmigen wasserabsorbierenden Polymeren enthaltenen thermoplastischen Klebstoffen in einer Menge von bis zu etwa 50 Gew.-%, vorzugsweise bis zu 60 Gew.-% und besonders bevorzugt bis zu 70 Gew.-%, jeweils bezogen auf den thermoplastischen Klebstoff, beispielsweise in einer Menge von etwa 0,1 bis etwa 35 Gew.-% oder etwa 3 bis etwa 20 Gew.-% enthalten sein.

Neben den bereits genannten Komponenten kann ein thermoplastischer Klebstoff, wie er in den erfindungsgemäßen pulverförmigen wasserabsorbierenden Polymeren enthalten ist, noch mindestens ein Wachs aufweisen. Unter einem "Wachs" wird im Rahmen der vorliegenden Erfindung eine nicht wasserlösliche Verbindung oder ein Gemisch aus zwei oder mehr solcher Verbindungen mit einem Schmelzpunkt von 90 bis etwa 165°C. Geeignete Wachse sind beispielsweise Paraffinwachse, Polyethylenwachse, Polypropylenwachse, Montanwachse, Fischer-Tropsch-Wachse, mikrokristalline Wachse oder Carnaubawachse.

Derartige Wachse können in einem thermoplastischen Klebstoff, wie er im Rahmen der vorliegenden Erfindung eingesetzt werden kann, in einer Menge von bis zu 60 Gew.-%, vorzugsweise bis zu 70 Gew.-% und besonders bevorzugt bis zu 80 Gew.-%, jeweils bezogen auf den thermoplastischen Klebstoff, beispielsweise in einer Menge von etwa 5 bis etwa 60 Gew.-%, vorliegen. Werden im Rahmen der vorliegenden Erfindung als thermoplastische Klebstoffe Verbindungen eingesetzt, die aus der Polymerisation von α-Olefinen erhältlich sind, so beträgt der Anteil an Wachsen in derartigen Schmelzklebstoffen vorzugsweise mindestens etwa 5 Gew.-% oder mindestens etwa 10 Gew.-%.

Es ist weiterhin in einer Ausgestaltung der vorliegenden Erfindung bevorzugt, dass der thermoplastische Klebstoff als flüssige Phase auf das pulverförmige wasserabsorbierende Polymer aufgebracht wird. Hierzu sind neben Lösungen des thermoplastischen Klebstoffs Emulsionen und Dispersionen geeignet. Als Lösemittel für diese flüssigen Phasen kommen alle dem Fachmann als geeignet bekannten organischen und anorganischen Lösemittel in Betracht. Die flüssigen Phasen beinhalten den thennoplastischen Klebstoff in einer Menge im Bereich von 0,001 bis 50 Gew.-%, vorzugsweise in einem Bereich von 0,01 bis 25 Gew.-% und besonders bevorzugt im Bereich von 0,1 bis 15 Gew.-%, jeweils bezogen auf die Lösemittelmenge. Besonders bevorzugt beinhaltet die flüssige Phase als Lösemittel zu mindestens 60 Gew.-%, vorzugsweise zu mindestens 80 Gew.-% Wasser. Weiterhin ist es bevorzugt, dass die flüssige Phase mindestens einen der nachfolgend beschriebenen Nachvernetzer aufweist, wobei in diesem Zusammenhang Nachvemetzermengen in einem Bereich von 0,001 bis 20 Gew.-% bevorzugt und in einem Bereich von 0,01 bis 10 Gew.-% besonders bevorzugt sind.

Unter den vorstehend offenbarten thermoplastischen Klebstoffen eignen sich sufonierte thermoplastische Klebstoffe besonders für die Verwendung in flüssigen Phasen. Hierunter sind sulfonierte Polyester, sulfonierte Polyamide wie Nylon, besonders bevorzugt. Eine weitere Gruppe von für flüssige Phasen geeignete thermoplastische Klebstoffe sind polyethoxilierte Polyamide wie Nylon beispielsweise erhältlich als Lurotex® der BASF AG in Ludwigshafen. Zudem eigenen sich für die Verwendung in flüssigen Phasen Styrol-Acrylsäure-Copolymere wie Joncryl® von SC Johnson, Blockcopolymere aufweisend Polyuerthan- und Polyethylenoxidblöcke, mindestens teilweise hydrolisierte Ethylenvinyacetat-copolymere und Poylacrylat und -Ester beinhaltende Emulsionen.

In einer bevorzugten Ausführungsform der erfindungsgemäßen pulvelförmigen wasserabsorbierenden Polymere basieren die in den Polymeren enthaltenen wasserabsorbierenden Polymerteilchen auf

| | |
|---|---|
| (α1) | 0,1 bis 99,999 Gew.-%, bevorzugt 20 bis 98,99 Gew.-% und besonders bevorzugt 30 bis 98,95 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind, |
| (α2) | 0 bis 70 Gew.-%, bevorzugt 1 bis 60 Gew.-% und besonders bevorzugt 1 bis 40 Gew.-% polymerisierten, ethylenisch ungesättigten, mit (α1) copolymerisierbaren Monomeren, |
| (α3) | 0,001 bis 10 Gew.-%, bevorzugt 0,01 bis 7 Gew.-% und besonders bevorzugt 0,05 bis 5 Gew.-% eines oder mehrerer Vernetzter, |
| (α4) | 0 bis 30 Gew.-%, bevorzugt 1 bis 20 Gew.-% und besonders bevorzugt 5 bis 10 Gew.-% wasserlöslichen Polymeren, sowie |
| (α5) | 0 bis 20 Gew.-%, bevorzugt 0,01 bis 7 Gew.-% und besonders bevorzugt 0,05 bis 5 Gew.-% eines oder mehrerer Hilfsstoffe, wobei die Summe der Gewichtsmengen (α1) bis (α5) 100 Gew.-% beträgt. |

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol%, besonders bevorzugt zu mindestens 50 Mol% und darüber hinaus bevorzugt zu 50-90 Mol% neutralisiert. Die Neutralisation der Monomere (α1) kann vor auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Aminogruppen, das im Vergleich zu dem sauren Polymer basisch ist, mindestens teilweise neutralisiert werden. Diese Polymere werden in der Literatur als ,Mixed-Bed Ion-Exchange Absorbent Polymers" (MBIEA-Polymere) bezeichnet und sind unter anderem in der WO 99/34843 offenbart. Die Offenbarung der WO 99/34843 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, ß-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, ß-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere (α1) des Weiteren ethylenisch ungesättigte Sülfonsäuremonomere oder ethylenisch ungesättigte Phosphonsäuremonomere bevorzugt.

Ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Styrolsulfonsäure bevorzugt. Als Acryl- bzw. Methacrylsulfonsäuren sind Sulfoethyl(meth)acrylat, Sulfopropyl(meth)acrylat und 2-Hydroxy-3-methacryloxypropylsulfonsäure bevorzugt. Als (Meth)acrylamidoalkylsulfonsäure ist 2-Acrylamido-2-methylpropansulfonsäure bevorzugt.

Ferner sind ethylenisch ungesättigte Phosphonsäuremonomere, wie Vinylphosphonsäure, Allylphosphonsäure, Vinylbenzylphosponsäure, (Meth)acrylamidoalkylphosphonsäuren, Acrylamidoalkyldiphosphonsäuren, phosphonomethylierte Vinylamine und (Meth)acrylphosphonsäurederivate bevorzugt.

Es ist erfindungsgemäß bevorzugt, dass das wasserabsorbierende Polymerteilchen zu mindestens 50 Gew.-%, vorzugsweise zu mindestens 70 Gew.-% und darüber hinaus bevorzugt zu mindestens 90 Gew.-% auf carboxylatgruppenhaltigen Monomeren besteht. Es ist erfindungsgemäß besonders bevorzugt, dass das wasserabsorbierende Polymerteilchen zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% neutralisiert ist.

Als ethylenisch ungesättigte, einen protonierten Stickstoff enthaltende Monomere (α1) sind vorzugsweise Dialkylaminoalkyl(meth)acrylate in protonierter Form, beispielsweise Dimethylaminoethyl(meth)acrylat-Hydrochlorid oder Dimethyla-minoethyl(meth)acrylat-Hydrosulfat, sowie Dialkylarninoalkyl(meth)acrylamide in protonierter Form, beispielsweise Dimethylaminoethyl(meth)acrylamid-Hydrochlorid, Diemethylaminopropyl(meth)acrylamid-Hydrochlorid, Diemethy-laminopropyl(meth)acrylamid-Hydrosulfat oder Dimethylaminoethyl(meth)acrylamid-Hydrosulfat bevorzugt.

Als ethylenisch ungesättigte, einen quarternierten Stickstoff enthaltende Monomere (α1) sind Dialkylammoniumalkyl(meth)acrylate in quarternisierter Form, beispielsweise Trimethylammoniumethyl(meth)acrylat-Methosulfat oder Dimethylethylammoniumethyl(meth)acrylat-Ethosulfat sowie (Meth)acrylamido-alkyldialkylamine in quarternisierter Form, beispielsweise (Meth)acrylamidopro-pyltrimethylammoniumchlorid, Trimethylammoniumethyl(meth)acrylat-Chlorid oder (Meth)acrylamidopropyltrimethylammoniumsulfat bevorzugt.

Als monoethylenisch ungesättigte, mit (α1) copolymerisierbare Monomere (α2) sind Acrylamide und Methacrylamide bevorzugt.

Mögliche (Meth)acrylamide sind neben Acrylamid und Methacrylamid alkyl-substituierte (Meth)acrylamide oder aminoalkylsubstituiene Derivate des (Meth)acrylamids, wie N-Methylol(meth)acrylamid, N,N-Dimethylamino(meth)-acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid. Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allylverbindungen, (Meth)acrylsäureester einer Monovinylverbindung oder (Meth)acrylsäureester einer Mono(meth)allylver-bindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen. Die Offenbarungen werden hiermit als Referenz eingeführt und gelten somit als Teil der Offenbarung.

Als Verbindungen der Vernetzerklasse I seien als Beispiel genannt Alkenyldi(meth)acrylate, beispielsweise Ethylenglykoldi(meth)acrylat, 1 ,3-Propylenglykoldi(meth)acrylat, 1,4-Butylenglylcoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopen-tandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Dihydroxyethylen)bis(meth)acrylamid, N,N'-Hexamethylenbis(meth)acrylamid oder N,N'-Methylenbis(meth)acrylamid, Polyalkoxydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropy-lenglykoldi(meth)acrylat oder Tetrapropylenglykoldi(meth)acrylat, Bisphenol-A-di(meth)acrylat, ethoxyliertes Bisphenol-A-di(meth)acrylat, Benzylidindi(meth)-acrylat, 1,3-Di(meth)acryloyloxy-propanol-2, Hydrochinondi(meth)acrylat, Di(meth)acrylatester des vorzugsweise mit 1 bis 30 Mol Alkylenoxid pro Hydroxylgruppe oxyalkylierten, vorzugsweise ethoxylierten Trimethylolpropans, Thi-oethylenglykoldi(meth)acrylat, Thiopropylenglykoldi(meth)acrylat, Thiopolyethy-lenglykoldi(meth)acrylat, Thiopolypropylenglykoldi(meth)acrylat, Divinylether, beispielsweise 1,4-Butandioldivinylether, Divinylester, beispielsweise Divinyla-dipat, Alkandiene, beispielsweise Butadien oder 1,6-Hexadien, Divinylbenzol, Di(meth)allylverbindungen, beispielsweise Di(meth)allylphthalat oder Di(meth)-allylsuccinat, Homo- und Copolymere von Di(meth)allyldimethylammoniumchlorid und Homo- und Copolymere von Diethyl(meth)allylaminomethyl(meth)acrylatammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri(meth)allylcyanurat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri(meth)allylphosphat Tetra(meth)allylethylendiamin, Poly(meth)allylester, Tetra(meth)allyloxiethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensationsvernetzer), in einer Additionsreaktion oder in einer Ringöffnungsreaktion mit den funktionellen Gruppen der Monomere (α1) oder (α2), bevorzugt mit Säuregruppen, der Monomeren (α1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat-oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polygly-cidylether-Verbindungen wie Ethylenglykoldiglycidylether, Polyethylenglykoldiglycidylether, Glycerindiglycidylether, Glycerinpolyglycidylether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbu-tanol-tris[3 -(1 -aziridinyl)propionat], 1,6-Hexamethylendiethylenharnstoff und Diphenylmethan-bis-4,4 '-N,N '-diethylenharnstoff, Halogenepoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat, sowie hydroxyl- oder aminogruppenhaltige (Meth)acrylamide, oder Mono(meth)allylverbindungen von Diolen bevorzugt.

Die polyvalenten Metallkationen der Vemetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ x 6H₂O, NaAl(SO₄)₂ * 12 H₂O, KAl(SO₄)₂ x 12 H₂O oder Al₂(SO₄)₃ x 14-18 H₂O eingesetzt.

Besonders bevorzugt werden Al₂(SO₄)₃ und seine Hydrate als Vernetzer der Vernetzungsklasse IV verwendet.

Bevorzugte wasserabsorbierende Polymerteilchen sind Polymerteilchen, die durch Vernetzer der folgenden Vernetzerklassen bzw. durch Vernetzer der folgenden Kombinationen von Vernetzerklassen vernetzt sind: I, II, III, IV, I II, I III, I IV, I II III, I II IV, I III IV, II III IV, II IV oder III IV. Die vorstehenden Kombinationen von Vernetzerklassen stellen jeweils eine bevorzugte Ausführungsform von Vernetzern eines wasserabsorbierenden Polymerteilchens dar.

Weitere bevorzugte Ausführungsformen der wasserabsorbierenden Polymerteilchen sind Polymerteilchen, die durch einen beliebigen der vorstehend genannten Vernetzer der Vernetzerklassen I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den wasserabsorbierenden Polymerteilchen wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) sind in den wasserabsorbierenden Polymerteilchen vorzugsweise Stellmittel, Geruchsbinder, oberflächenaktive Mittel oder Antioxidatien enthalten.

Die in den erfindungsgemäßen pulverförmigen, wasserabsorbierenden Polymeren enthaltenen vorstehend beschriebenen Polymerteilchen sind vorzugsweise durch Massepolymerisation, die vorzugsweise in Knetreaktoren wie Extrudern oder durch Bandpolymerisation erfolgt, durch Lösungspolymerisation, Spraypolymerisation, inverse Emulsionspolymerisation oder inverse Suspensionspolymerisation erhältlich. Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Um die durch die vorstehend genannten Polymerisationsverfahren erhaltenen wasserabsorbierenden Polymere in eine partikuläre Form mit einer mittleren Partikelgröße von mindestens 100 µm, vorzugsweise von mindestens 150 µm und besonders bevorzugt von mindestens 200 µm zu überführen, können diese nach ihrer Abtrennung aus der Reaktionsmischung zunächst bei einer Temperatur in einem Bereich von 20 bis 300°C, bevorzugt in einem Bereich von 50 bis 250°C und besonders bevorzugt in einem Bereich von 100 bis 200°C bis hin zu einem Wassergehalt von weniger als 40 Gew.-%, bevorzugt von weniger als 20 Gew.-% und darüber hinaus bevorzugt von weniger als 10 Gew.-%, jeweils bezogen auf das Gesamtgewicht des Polymers, getrocknet werden. Die Trocknung erfolgt vorzugsweise in dem Fachmann bekannten Öfen oder Trocknern, beispielsweise in Bandtrocknern, Hordentrocknern, Drehrohröfen, Wirbelbetttrocknern, Tellertrocknem, Paddeltrocknern oder Infrarottrocknern. Sollten die so erhaltenen, getrockneten Polymere noch nicht in partikulärer Form vorliegen, so müssen sie nach der Trocknung noch zerkleinert werden. Das Zerkleinern erfolgt dabei vorzugsweise durch Trockenmahlen, vorzugsweise durch Trockenmahlen in einer Hammermühle, einer Stiftmühle, einer Kugelmühle oder einer Walzenmühle.

Neben dem vorstehend beschriebenen Verfahren zur Überführung des Polymers in eine partikuläre Form können die Polymere auch im gelförmigen Zustand durch das Verfahren des Nassmahlens mit einer beliebigen, konventionellen Vorrichtung zum Nassmahlen zerkleinert werden.

In einer anderen bevorzugten Ausführungsform basieren die in den pulverförmigen wasserabsorbierenden Polymeren enthaltenen wasserabsorbierenden Polymerteilchen auf biologisch abbaubaren Materialien. Dazu gehören natürliche Polysaccharide, die bereits von natur aus Carboxylgruppen aufweisen oder die durch nachträgliche Modifizierung mit Carboxylgruppen versehen worden sind. Zur ersten Gruppe von Polysacchariden zählen beispielsweise Stärke, Amylose, Amylopektin, Cellulose und Polygalaktomannane wie Guar und Johannesbrotkernmehl, zur zweiten Gruppe zählen beispielsweise, Xanthan, Alginate, Gummi Arabicum.

In einer weiteren bevorzugten Ausführungsform basieren die in den pulverkrmigen wasserabsorbierenden Polymeren enthaltenen wasserabsorbierenden Polymerteilchen auf einer Mischung aus biologisch abbaubaren und synthetischen wasserabsorbierenden Polymerteilchen.

Es ist weiterhin erfindungsgemäß bevorzugt, dass die wasserabsorbierenden Polymerteilchen einen Innenbereich, einen den Innenbereich umgebenden Außenbereich sowie einen den Außenbereich umgebenden Oberflächenbereich aufweisen, wobei der Außenbereich einen höheren Vernetzungsgrad als der Innenbereich aufweist, so dass sich vorzugsweise eine Kern-Schale-Struktur ausbildet. Die erhöhte Vernetzung im Oberflächenbereich der pulverförmigen wasserabsorbierenden Polymere wird dabei vorzugsweise durch Nachvernetzung oberflächennaher, reaktiver Gruppen erreicht. Diese Nachvernetzung kann thermisch, photochemisch oder chemisch erfolgen. Als Nachvernetzer für die chemische Nachvernetzung sind dabei die Verbindungen bevorzugt, die als Vernetzer (α3) der Vernetzerklassen II und IV genannt wurden. Besonders bevorzugt als Nachvernetzer ist Ethylencarbonat.

Bei dem enfindungsgemäßen Verfahren zur Herstellung pulverförmiger wasserabsorbierender Polymere werden als Bestandteile:
- 0,01 bis 20 Gew.-% vorzugsweise 0,1 bis 15 Gew.-% und darüber hinaus bevorzugt 1 bis 10 Gew.-%, eines vorzugsweise nicht auf wasserabsorbierenden, vernetzten Poly(meth)acrylaten basierenden Feinteilchens mit einer durch Siebanalyse bestimmten mittleren Teilchengröße von weniger als 200 µm, vorzugsweise von weniger als 100 µm und besonders bevorzugt von weniger als 50 µm;
- 0,001 bis 10 Gew.-%, vorzugsweise 0,01 bis 5 Gew.-% und besonders bevorzugt 0,5 bis 1 Gew.-% eines thermoplastischen Klebstoffs,
- 60 bis 99,998 Gew.-%, vorzugsweise 70 bis 99 Gew.-% und besonders bevorzugt 90 bis 95 Gew.-% eines wasserabsorbierenden Polymerteilchens mit einer durch Siebanalyse bestimmten mittleren Teilchengröße von mindestens 200 um, vorzugsweise von mindestens 250 µm und besonders bevorzugt von mindestens 300 µm, wobei die Summe der vorstehenden Komponenten 100 Gew.-% beträgt,
bei einer Temperatur im Bereich von 120 bis 250 °C, bevorzugt 150 bis 220 °C und besonders bevorzugt 170 bis 200 °C miteinander in Kontakt gebracht.

In einer Ausgestaltung des vorstehenden Verfahrens wird als weitere Bestandteil noch mindestens einer der vorstehend definierten Nachvernetzer eingesetzt. In diesem Fall ist es bevorzugt, dass das wasserabsorbierende Feinteilchen weicht nach- bzw. oberflächenvernetzt ist. Weiterhin ist es in dieser Ausgestaltung bevorzugt, dass thermoplastischer Klebstoff und Nachvernetzer in einer flüssigen Phase, vorzugsweise in einer wässrigen Lösung eingesetzt werden, wobei die vorstehend im Zusammenhang mit den flüssigen Phasen angegebenen Konzentrationen auch hier bevorzugt sind. In diesem Zusammenhang ist es bevorzugt, dass als weiterer Bestandteil ein Nachvernetzer in Kontakt gebracht wird. Besonders bevorzugt ist hierbei, dass der Nachvernetzer und der thermoplastische Klebstoff gemeinsam den anderen Bestandteilen zugeführt werden, wobei mindestens der Nachvernetzer in einer flüssigen Phase vorliegen kann.

Als wasserabsorbierende Polymerteilchen, thermoplastische Klebstoffe und Feinteilchen sind dabei diejenigen wasserabsorbierenden Polymerteilchen, thermoplastischen Klebstoffe oder Feinteilchen bevorzugt, die bereits im Zusammenhang mit den erfindungsgemäßen pulverförmigen wasserabsorbierenden Polymeren genannt wurden.

Es ist erfindungsgemäß bevorzugt, dass die thermoplastischen Klebstoffe in partikuläre Form, vorzugsweise in Form eines Pulvers oder eines Granulates eingesetzt werden, wobei mindestens 50 Gew.-%, vorzugsweise mindestens 70 Gew.-% des Pulvers oder Granulats eine nach Siebanalyse bestimmte Partikelgröße in einem Bereich von 1 bis 200 um, besonders bevorzugt in einem Bereich von 10 bis 150 µm und darüber hinaus bevorzugt in einem Bereich von 20 bis 120 µm.

Sollten die im Zusammenhang mit den erfindungsgemäßen pulverförmigen wasserabsorbierenden Polymere genannten thermoplastischen Klebstoffe nach ihrer Herstellung oder bei ihrer Lieferung nicht in partikulärer Form, sondern beispielsweise in Form von Blöcken vorliegen, so können sie durch dem Fachmann bekannte Zerkleinerungsverfahren, wie beispielsweise durch Mahlen oder durch Extrudieren durch Lochscheiben und anschließendes Zerkleinern der extrudierten Polymere in eine partikuläre Form mit der erforderlichen mittleren Partikelgröße überführt werden. Gegebenenfalls können besondere Mahlverfahren eingesetzt werden, beispielsweise die Cryovermahlung bei tiefen Temperaturen, insbesondere bei Temperaturen unterhalb der Schmelz- oder Glasübergangstemperatur der eingesetzten thermoplastischen Klebstoffe. Eine wichtige Voraussetzung für das Mahlen zu der gewünschten Korngröße ist die Sprödigkeit des Schmelzklebers.

Das Zusammenbringen von wasserabsorbierenden Polymerteilchen, thermoplastischen Klebstoffe und Feinteilchen kann auf beliebige Weise erfolgen. Voraussetzung zur Herstellung der erfindungsgemäßen pulverförmigen wasserabsorbierenden Polymere ist jedoch, dass zumindest eine Kontaktzeit zwischen wasserabsorbierenden Polymerteilchen, thermoplastischem Klebstoff und Feinteilchen eingehalten wird, die eine durch den thermoplastischen Klebstoff vermittelte Klebeverbindung zwischen mindestens einem wasserabsorbierenden Polymerteilchen und mindestens einem Feinteilchen erlaubt. Vorzugsweise wird die Kontaktzeit so bemessen, dass möglichst kleine Aggregate entstehen, die Idealerweise jeweils nur ein wasserabsorbierendes Polymerteilchen und ein oder mehrere durch den thermoplastischen Klebstoff auf der Oberfläche des wasserabsorbierenden Polymerteilchens aufgebrachte Feinteilchen umfassen. Es soll vorzugsweise vermieden werden, dass sich durch zu lange Kontaktzeiten aus mehreren wasserabsorbierenden Polymerteilchen bestehende Agglomerate durch die Klebewirkung des thermoplastischen Klebstoffes bilden.

Das Zusammenbringen der wasserabsorbierenden Polymerteilchen, des thermoplastischen Klebstoffs und der Feinteilchen kann im Rahmen des erfindungsgemäßen Verfahrens auf beliebige Weise erfolgen. So können beispielsweise die thermoplastischen Klebstoffe in partikulärer Form sowie die Feinteilchen mit den wasserabsorbierenden Polymerteilchen bereits während des Herstellungsprozesses für die wasserabsorbierenden Polymerteilchen in Kontakt gebracht werden. Geeignet ist hierzu beispielsweise ein Kontaktieren der wasserabsorbierenden Polymerteilchen mit den thermoplastischen Klebstoffpartikeln und den Feinteilchen im Rahmen einer Nachvernetzung des wasserabsorbierenden Polymerteilchens, wie sie üblicherweise bei der Herstellung von Superabsorbern durchgeführt wird.

Hinsichtlich der Reihenfolge des in Kontakt bringens der einzelnen Komponenten sind verschiedene Vorgehensweisen möglich.

In einer Ausführungsform des erfindungsgemäßen Verfahrens werden zunächst die wasserabsorbierenden Polymerteilchen mit dem partikulären thermoplastischen Klebstoff in fester Form vermischt. Die auf diese Weise mit dem thermoplastischen Klebstoff in Kontakt gebrachten wasserabsorbierenden Polymerteilchen können anschließend mit den Feinteilchen vermischt werden. Um eine durch den thermoplastischen Klebstoff vermitteltes Anheften der Feinteilchen auf der Oberfläche der wasserabsorbierenden Polymerfeinteilchen zu bewirken, muss der thermoplastische Klebstoff durch eine Temperaturerhöhung zunächst auf eine Temperatur oberhalb der Schmelz- oder Glasübergangstemperatur erhitzt werden, so dass er ein klebriges Verhalten zeigt. Dabei kann das Erhitzen zu einem Zeitpunkt erfolgen, in dem die wasserabsorbierenden Polymerteilchen mit dem thermoplastischen Klebstoff, aber noch nicht mit den Feinteilchen in Kontakt gebracht worden sind. In diesem Fall wird die Mischung aus den Polymerfeinteilchen und dem thermoplastischen Klebstoff auf eine Temperatur oberhalb der Schmelztemperatur des thermoplastischen Klebstoffes entsprechend erhitzt und diese Mischung anschließend mit den Feinteilchen in Kontakt gebracht. Um eine unerwünschte Agglomeration der wasserabsorbierenden Polymerteilchen zu vermeiden, kann es dabei von Vorteil sein, die mit dem Klebstoff in Kontakt gebrachten Polymerteilchen vorübergehend auf eine Temperatur unterhalb der Schmelztemperatur des thermoplastischen Klebstoffes abzukühlen. Während dieser Abkühlphase sollten die wasserabsorbierenden Polymerteilchen bewegt werden. Eine derartige Bewegung kann beispielsweise durch Rühren, Schütteln oder Fließen oder dergleichen verursacht werden. Nach dem in Kontakt bringen der mit dem thermoplastischen Klebstoff beschichteten wasserabsorbierenden Polymerteilchen mit den Feinteilchen muss die Temperatur wieder auf eine Temperatur oberhalb der Schmelz- oder Glasübergangstemperatur des thermoplastischen Klebstoffes erhöht werden, um eine Haftvermittlung zwischen den wasserabsorbierenden Polymerteilchen und den Feinteilchen sicherzustellen. Zudem können in einer anderen Ausgestaltung des erfindungsgemäßen Verfahrens die wasserabsorbierenden Polymerteilchen zunächst mit den Feinteilchen vermischt und anschließend der thermoplastische Klebstoff zugegeben werden, worauf die Erwärmung zum Schmelzen des thermoplastischen Klebstoffs folgt. So kann eine homogenere Mischung der Feinteilchen mit den wasserabsorbierenden Polymerteilchen erhalten werden, was zu weniger Staubbildung und einer verbesserten Fließfahigkeit führt.

Möglich ist aber auch, den thermoplastischen Klebstoff zu einem Zeitpunkt zu erhitzen, in dem er noch nicht mit den wasserabsorbierenden Polymerteilchen in Kontakt gebracht wurde. In diesem Fall wird zunächst der thermoplastische Klebstoff auf eine Temperatur oberhalb der Schmelz- oder Glasübergangstemperatur des thermoplastischen Klebstoffes erhitzt und anschließend mit den wasserabsorbierenden Polymerteilchen in Kontakt gebracht. Die auf diese Weise mit dem thermoplastischen Klebstoff in Kontakt gebrachten wasserabsorbierenden Polymerteilchen werden anschließend mit dem Feinteilchen vermischt, wobei auch hier zur Vermeidung der Bildung größerer Agglomerate eine zwischenzeitige Abkühlung erfolgen kann.

In einer weiteren Ausführungsform des erfindungsgemäßen Verfahrens wird wie vorstehend verfahren, mit dem Unterschied, dass zunächst die Feinteilchen mit dem partikulären thermoplastischen Klebstoff in fester Form vermischt werden und anschließend die mit dem thermoplastischen Klebstoff in Kontakt gebrachten Feinteilchen mit anschließend mit den wasserabsorbierenden Polymerteilchen vermischt werden.

In einer besonders bevorzugten Ausführungsform des erfindungsgemäßen Verfahrens jedoch werden die Feinteilchen, die wasserabsorbierenden Polymerteilchen und der thermoplastische Klebstoff zeitgleich miteinander in Kontakt gebracht. Dabei kann der thermoplastische Klebstoff in partikulärer Form bei einer Temperatur unterhalb der Schmelztemperatur mit den wasserabsorbierenden Polymerteilchen und den Feinteilchen vermischt und die so erhaltene Mischung anschließend auf eine Temperatur oberhalb der Schmelztemperatur des thennoplastischen Klebstoffes erhitzt werden, um die Haftvermittlung durch den thermoplastischen Klebstoff zwischen den Feinteilchen und den wasserabsorbierenden Polymerteilchen sicherzustellen. Möglich ist auch, den thermoplastischen Klebstoff zunächst auf eine Temperatur oberhalb der Schmelztemperatur zu erhitzen und den so erhitzten thermoplastischen Klebstoff zusammen mit den Feinteilchen und den wasserabsorbierenden Polymerteilchen zu vermischen. Auch in dieser Ausführungsform des erfindungsgemäßen Verfahrens kann es sinnvoll sein, zur Vermeidung einer unerwünschten Agglomeration der wasserabsorbierenden Polymerteilchen die mit dem Klebstoff in Kontakt gebrachten Polymerteilchen vorübergehend auf eine Temperatur unterhalb der Schmelz- oder Glasübergangstemperatur des thermoplastischen Klebstoffes abzukühlen. Während dieser Abkühlphase sollten die wasserabsorbierenden Polymerteilchen bewegt werden.

Das in Kontakt bringen der wasserabsorbierenden Polymerteilchen mit dem thermoplastischen Klebstoff und den Feinteilchen kann in dem Fachmann bekannten Mischaggregaten erfolgen. Geeignete Mischaggregate Komponenten sind z. B. der Patterson-Kelley-Mischer, DRAIS-Turbulenzmischer, Lödigemischer, Ruberg-Mischer, Schneckemnischer, Tellermischer und Wirbelschichtmischer sowie kontinuierlich fördernde, vorzugsweise senkrechte Mischöfen, in denen die Partikel mittels rotierender Messer in schneller Frequenz gemischt wird und die bewegliche, beheizte Fördermittel aufweisen.

Es ist erfindungsgemäß bevorzugt, wenn die pulverförmigen wasserabsorbierenden Polymere in einem weiteren Verfahrensschritt im Oberflächenbereich nachvemetzt werden, wobei diejenigen Nachvernetzungsmittel bevorzugt sind, die bereits im Zusammenhang mit den erfindungsgemäßen pulverförmigen wasserabsorbierenden Polymeren als bevorzugte Nachvernetzer genannt wurden.

Für den Zeitpunkt der Nachvernetzung während des erfindungsgemäßen Verfahrens gibt es mehrere Möglichkeiten.

In einer Ausführungsform des erfindungsgemäßen Verfahrens erfolgt die Nachvernetzung, bevor die wasserabsorbierenden Polymerteilchen mit dem thermoplastischen Klebstoff und den Feinteilchen in Kontakt gebracht wurden. In diesem Fall werden die Polymerfeinteilchen mit dem Nachvernetzer, vorzugsweise mit einem Fluid beinhaltend ein Lösungsmittel, vorzugsweise Wasser, organische Lösungsmittel wie Methanol, Ethanol, 1-Propanol oder 2-Propanol, oder eine Mischung aus mindestens zwei davon, und den Nachvernetzer, in Kontakt gebracht. Anschließend werden die mit dem Nachvernetzer in Kontakt gebrachten wasserabsorbierenden Polymerteilchen auf eine Temperatur in einem Bereich von 50 bis 300°C, vorzugsweise in einem Bereich von 100 bis 250°C und besonders bevorzugt in einem Bereich von 150 bis 200°C erhitzt, um die Nachvernetzung durchzuführen. Anschließend werden die so nachvernetzten Polymerteilchen in der vorstehend beschriebenen Weise mit dem thermoplastischen Klebstoff und den Feinteilchen in Kontakt gebracht.

In einer anderen Ausführungsform des erfindungsgemäßen Verfahrens erfolgt die Nachvernetzung, nachdem die wasserabsorbierenden Polymerteilchen mit dem thermoplastischen Klebstoff und den Feinteilchen in Kontakt gebracht wurden. In diesem Fall werden die pulverförmigen wasserabsorbierenden Polymere, in dessen Oberflächenbereich die Feinteilchen mittels des thermoplastischen Klebstoffs immobilisiert sind, in der vorstehend beschriebenen Art und Weise nachvernetzt.

In einer bevorzugten Ausführungsform des erfindungsgemäßen Verfahrens erfolgt die Nachvernetzung während des in Kontakt bringens der wasserabsorbierenden Polymerteilchen mit dem thermoplastischen Klebstoff und den Feinteilchen. Dabei ist es besonders bevorzugt, dass die wasserabsorbierenden Polymerteilchen mit dem vorzugsweise partikulären thermoplastischen Klebstoff, den Feinteilchen und dem Nachvernetzer, vorzugsweise dem Nachvernetzer in Form eines Fluids umfassend den Nachvernetzer und ein Lösungsmittel, vermischt und anschließend auf eine Temperatur oberhalb der Schmelztemperatur erhitzt werden.

Vorzugsweise wird der Nachvernetzer in einer Menge in einem Bereich von 0,01 bis 5 Gew.-%, besonders bevorzugt in einem Bereich von 0,3 bis 3 Gew.-% und darüber hinaus bevorzugt in einer Menge in einem Bereich von 0,5 bis 1,5 Gew.-%, jeweils bezogen auf das Gewicht der wasserabsorbierenden Polymerteilchen, eingesetzt. Wird der Nachvernetzer in Form eines Fluids, vorzugsweise in Form einer wässrigen Lösung oder Dispersion eingesetzt, so enthält dieses Fluid den Nachvernetzer vorzugsweise in einer Menge in einem Bereich von 10 bis 80 Gew.-%, besonders bevorzugt in einer Menge in einem Bereich von 30 bis 70 Gew.-% und darüber hinaus bevorzugt in einer Menge in einem Bereich von 40 bis 50 Gew.-%, jeweils bezogen auf das Gesamtgewicht des Fluids.

Die Erfindung betrifft auch die durch das vorstehend beschriebene Verfahren erhältlichen pulverförmigen wasserabsorbierenden Polymere, wobei diese pulverförmigen wasserabsorbierenden Polymere vorzugsweise die gleichen Eigenschaften aufweisen wie die eingangs beschriebenen, erfindungsgemäßen pulverförmigen wasserabsorbierenden Polymere.

Besonders bevorzugt sind die durch das erfindungsgemäße Verfahren erhältlichen pulverförmigen wasserabsorbierenden Polymere durch

| | |
|---|---|
| P1 | einen Fließ-Wert (FFC) im Bereich von 1 bis 13 vorzugsweise im Bereich von 3 bis 9 und besonders bevorzugt im Bereich von 4 bis 8 und darüber hinaus bevorzugt im Bereich von 5 bis 7; oder |
| P2 | einen Staubanteil von höchstens 6, vorzugsweise höchstens 4, besonders bevorzugt höchstens 2, |

gekennzeichnet.

Es ist erfindungsgemäß weiterhin bevorzugt, dass die erfindungsgemäßen pulverförmigen wasserabsorbierenden Polymere und die durch das erfindungsgemäße Verfahren erhältlichen pulverförmigen wasserabsorbierenden Polymere mindestens eine, vorzugsweise jede, der folgenden Eigenschaften aufweisen:

| | |
|---|---|
| P3 | einen Attrition-Index Aᵢ im Bereich von 1 bis 17, vorzugsweise im Bereich von 1,1 bis 15 und besonders bevorzugt im Bereich von 1,5 bis 10; |
| P4 | eine Attrition-Differenz A_{d} im Bereich von 0 bis 7, vorzugsweise im Bereich von 1,1 bis 6 und besonderes bevorzugt im Bereich von 1,5 bis 3; |
| P5 | eine Retention bestimmt nach ERT 441.1-99 von mindestens 20 g/g, vorzugsweise .mindestens 25 g/g und besonders bevorzugt mindestens 27 g/g sowie darüber hinaus bevorzugt im Bereich von 27 bis 35 g/g. |

Die sich aus den vorstehenden Eigenschaften P1 bis P5 ergebenden Eigenschaftskombinationen von zwei oder mehr dieser Eigenschaften stellen jeweils bevorzugte Ausführungsformen der vorliegenden Erfindung dar. Weiterhin bevorzugt sind die erfindungsgemäßen pulverförmigen wasserabsorbierenden Polymere mit nachfolgend als Ziffer oder Zifferkombinationen dargestellten Eigenschaften oder' Eigenschaftskombinationen: P1P3P4P5, P1P5, P1P3P5, P3P5 oder P1P2P3P4P5.

In einer Ausführungsform betrifft die Erfindung ein pulverförmiges wasserabsorbierendendes Polymer, beinhaltend zu mindestens 30 Gew.-%, vorzugsweise mindestens 75 Gew.-% und besonders bevorzugt mindestens 95 Gew.-% einer vernetzten, teilneutralisierten, vorzugsweise im Bereich von 60 bis 80 Mol-% neutralisierten, Polyacrylsäure und mit mindestens einer, vorzugsweise jeder, der Eigenschaften P1 bis P5, vorzugsweise der Eigenschaftskombinationen P1P3P4P5, P1P5, P1P3P5 und darüber hinaus bevorzugt die Eigenschaftskombination P3P5.

Es ist weiterhin bevorzugt, dass mindestens 50 Gew.-%, besonders bevorzugt mindestens 70 Gew.-% und darüber hinaus bevorzugt mindestens 90 Gew.-% der durch das erfindungsgemäße Verfahren erhältlichen pulverförmigen wasserabsorbierenden Polymere sowie der erfindungsgemäßen pulverförmigen wasserabsorbierenden Polymere eine durch Siebanalyse bestimmte Teilchengröße im Bereich von größer 50 bis 2.000 um, bevorzugt in einem Bereich von 100 bis 1.500 µm und darüber hinaus bevorzugt in einem Bereich von 200 bis 1.200 µm besitzen. Bei dem erfindungsgemäßen Transportverfahren durchströmen die durch das erfindungsgemäße Verfahren erhältlichen pulverförmigen wasserabsorbierenden Polymere oder die erfindungsgemäßen pulverförmigen wasserabsorbierenden Polymere eine Röhre, wobei die Röhre vorzugsweise einen Teil einer Anlage zur Herstellung eines wasserabsorbierenden Polymers oder eines Dosiersystems für ein wasserabsorbierendes Polymer bildet. Derartige Dosiersysteme können beispielsweise beim Beladen von Containern oder Säcken verwand werden oder finden sich in Airlaid- oder Windelmaschinen. Besonders bevorzugt handelt es sich bei der Röhre um diejenige Röhre, in der die pulverförmigen wasserabsorbierenden Polymere geleitet werden, wenn sie mit einem faserförmigen Material, wie beispielsweise Baumwollfasern, in Kontakt unter Bildung eines absorbierenden Cores bzw. Kerns gebracht werden.

Der erfindungsgemäße Verbund beinhaltend ein zuvor definiertes pulverförmiges wasserabsorbierendes Polymere und ein Substrat. Vorzugsweise sind das erfindungsgemäße pulverförmige wasserabsorbierende Polymer und das Substrat fest miteinander verbunden. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt.

Erfindungsgemäß sind als Verbund Dichtmaterialien, Kabel, absorbierende Cores sowie diese enthaltende Windeln und Hygieneartikel bevorzugt.

Bei dem erfindungsgemäßen Verfahren zur Herstellung eines Verbunds werden ein erfindungsgemäßes pulverförmiges wasserabsorbierendes Polymer oder ein durch das erfindungsgemäße Verfahren erhältliches pulverförmiges wasserabsorbierendes Polymer und ein Substrat und ggf. ein geeignetes Hilfsmittel miteinander in Kontakt gebracht werden. Das in Kontakt bringen erfolgt vorzugsweise durch Wetlaid- und Airlaid-Verfahren, Kompaktieren, Extrudieren und Mischen.

Zudem betrifft die Erfindung ein Verbund, der durch das vorstehende Verfahren erhältlich ist.

Ferner betrifft die Erfindung chemische Produkte, insbesondere Schäume, Formkörper, Fasern, Folien, Filme, Kabel, Dichtungsmaterialien, flüssigkeitsaufnehmende Hygieneartikel, Träger für pflanzen- oder pilzwachstumsregulierende Mittel oder Pflanzenschutzwirkstoffen, Zusätze für Baustoffe, Verpackungsmaterialien oder Bodenzusätze, die das erfindungsgemäße pulverförmige wasserabsorbierende Polymer oder das durch das erfindungsgemäße Verfahren erhältliche pulverförmige wasserabsorbierende Polymer oder den vorstehend beschriebenen Verbund beinhalten. Diese chemischen Produkte zeichnen sich insbesondere durch eine besonders gute biologische Abbaubarkeit aus.

Außerdem betrifft die Erfindung die Verwendung des erfindungsgemäßen pulverförmigen wasserabsorbierenden Polymers oder des durch das erfindungsgemäße Verfahren erhältlichen pulverförmigen wasserabsorbierenden Polymers oder des zuvor beschriebenen Verbundes in Hygieneprodukten, zur Hochwasserbekämpfung, zur Isolierung gegen Wasser, zur Regulierung des Wasserhaushalts von Böden oder zur Behandlung von Lebensmitteln.

Schließlich betrifft die Erfindung auch die Verwendung eines thermoplastischen Klebstoffes zur Veränderung des Fließ-Wertes (FFC) oder des Staubanteils bei diesen thermoplastischen Klebstoff beinhaltenden pulverförmigen wasserabsorbierenden Polymeren gegenüber diesen thermoplastischen Klebstoff nicht beinhaltenden pulverförmigen wasserabsorbierenden Polymeren, wobei als thermoplastischer Klebstoff diejenigen Klebstoffe bevorzugt sind, die bereits im Zusammenhang mit den erfindungsgemäßen pulverförmigen wasserabsorbierenden Klebstoffen genannt wurden.

Die Erfindung wird nun anhand von Testmethoden und nicht limitierenden Beispielen näher erläutert.

### TESTMETHODEN

### BESTIMMUNG DES FFC-WERTES

Der FFC-Wert gibt Auskunft über die Fließeigenschaften eines Schüttgutes in einem Silo. Bei der Messung wird das Schüttgut verschiedenen Belastungen ausgesetzt. Das Fließverhalten lässt sich wie folgt charakterisieren;

| | |
|---|---|
| FFC < 1 | nicht fließend |
| 1 < FFC < 2 | sehr kohäsiv |
| 2 < FFC < 4 | kohäsiv |
| 4 < FFC < 10 | leicht fließend |
| 10 < FFC | frei fließend |

Gutes Fließverhalten liegt .dann vor, wenn ein Schüttgut ohne großen Aufwand zum Fließen zu bringen ist, z. B. wenn das Schüttgut ohne Verfestigung aus einem Trichter oder einem Silo ausläuft. Bei schlecht fließenden Schüttgütern kommt es zu Auslaufstörungen oder sie verfestigen sich während des Transports oder der Lagerung. Mit dem Begriff "Fließen" ist gemeint, dass sich das Schüttgut aufgrund von Belastung plastisch verformt.

Weitere Angaben zu der genauen Durchführung des Tests zur Bestimmung des FFC sind den Artikeln von Herrn Dr. Ing. Dietmar Schulze "Das automatische Ringschergerät RST-01.pc" aus dem Februar 2002 und "Fließeigenschaften von Schüttgütern und verfahrenstechnische Siloauslegung" aus dem Jahr 2002 zu entnehmen. Bei dem vorliegen Messungen wurde die manuell betriebene Variante des Ringschergeräts RST-01.01 verwendet.

### BESTIMMUNG DES STAUBANTEILS

Der Staubanteil mit einem Gerät der Firma Palas, Deutschland des Typs "Dust View" bestimmt. Hierzu wird eine Probe von 30,00 g in ein Trichterrohr gegeben. Zu Beginn der Messung öffnet sich eine Trichterklappe automatisch und die Probe fällt in ein Staubreservoir. Nun wird die Verringerung eines Laserstrahls (Abnahme der Transmission) durch die Staubbildung gemessen. Dieser Wert dient der Bestimmung des Staubanteils, d.h. der Trübung, in Prozent mit einer Skala von 1 bis 100. Der Staubanteil ergibt sich aus einem Startwert am Beginn der Messung und einem nach 30 Sekunden gemessenen Staubwert zur Bestimmung des schwebenden Anteils. Somit ergibt sich der Staubanteil aus der Summe aus Startwert und Staubwert.

### BESTIMMUNG DES ATTRITION-INDEX UND DER ATTRITION-DIFFERENZ

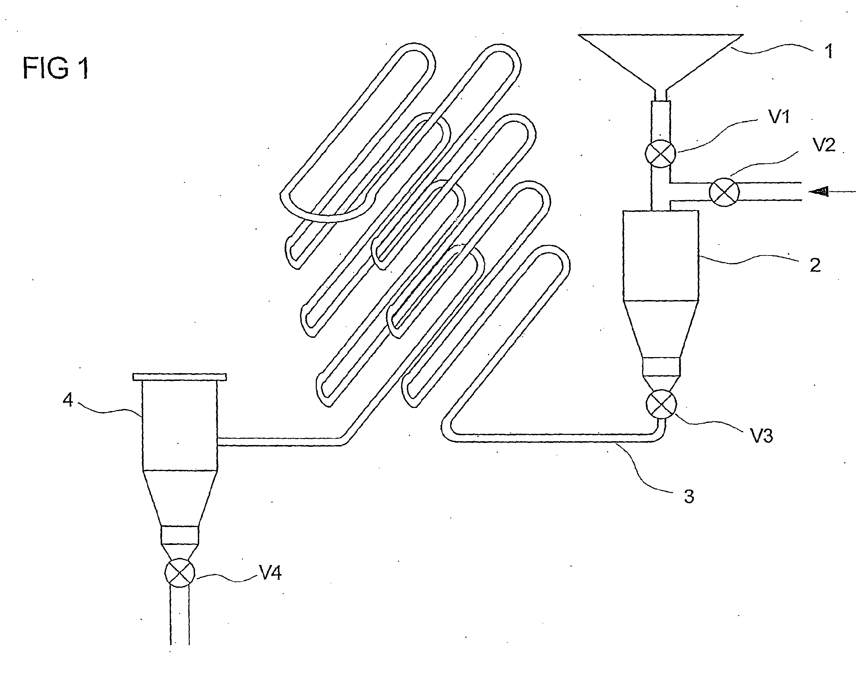

Hierzu wird die in Fig. 1 dargestellte Testvorrichtung eingesetzt. Diese besteht aus einem Trichter 1 zum Einführen einer Probe in eine Probenkammer 2. Zwischen dem Trichter und der Probenaufnahmekammer 2 ist eine Leitung mit einem Ladeventil V1 und einer unter dem Ladeventil V1 angeordneten mit einem Gasregulationsventil V2 versehenen Zuleitung vorgesehen. Über V2 wird ein Druck von 4,3 bar auf die Probenaufnahmekammer 2 aufgebracht. Diese besteht aus einem Zylinder mit einem Durchmesser von 9 cm und einer Länge von 16 cm. An diesen Zylinder schließt sich ein stumpfkegelförmiger Unterbereich an, der eine untere Öffnung mit einem Durchmesser von 4 cm besitzt. Daran schließt sich ein 2 cm langes Rohr mit einem Durchmesser von 4 cm an, das wiederum in einen weiteren stumpfkegelförmigen Durchlauf mit einer Länge von 4,5 cm und einem unteren Durchmesser von 1,75 cm mündet. Hieran schließt sich ein Kammerauslassventil V3 an. Auf V3 folgt über ein Kurvenstück von 90° von 4,5 cm Länge eine Rohrleitung mit einem Innendurchmesser von 10 mm und einer Gesamtlänge von 1101 cm. Die Rohrleitung beginnt mit einem geraden Abschnitt von 30 cm, hierauf folgen mit Kurvenstück von 90° von 4,5 cm Länge verbundene 16 gerade Abschnitte mit einer Länge von je 60 cm, die über 180° Kurvenabschnitte von jeweils 4,5 cm miteinander verbunden sind. An die 16 Abschnitte schließt sich ein weiterer gerader Abschnitt von 30 cm Länge über ein Kurvenstück von 90° und 4,5 cm Länge an. Dieser Abschnitt mündet in eine Probensammelkammer 4, die sich in ihren Aufbau von der Kammer 2 nur dadurch unterscheidet, dass anstelle eines Oberdeckels ein Filterbeutel mit einer im Vergleich zu den Teilchengrößen der Proben deutlich kleineren Porengröße angebracht ist. Die Probensammelkammer wird unten durch ein Probenentnahmeventil V4 abgeschlossen. Alle Metallteile der vorstehend beschriebenen Testvorrichtung sind aus 316Alloy Saines Steal gefertigt. Bei den Rohren handelt es sich um Stand. Stock Pipes. Die Innenflächen der einzelnen Komponenten der Vorrichtung sind glatt. Ventile des Typs 20SS0R-02-LL-9102 von der Firma Milwaukee Valve Company, USA wurden eingesetzt.

Die vorstehend beschriebene Vorrichtung wird wie folgt betrieben:

### Schritt 1 - Probenbeschickung

Die Ventile V1, V4 sind offen, die Ventile V2 und V3 sind geschlossen. 50 g einer Probe wird über den Trichter 1 in die Kammer 2 eingefüllt.

### Schritt 2 - Probentransport

Die Ventile werden in folgender Reihenfolge bedient: a) V1 schließen, b) V2 öffnen, c) V3 schließen. Nachdem die Rohrleitung keine durch den Probendurchgang bedingten Schwingungen mehr zeigt, sind folgenden Ventilstellungen einzustellen: a) V2 schließen, V1 öffnen, V3 schließen.

### Schritt 3 - Probenentnahme

Zur Probenentnahme wird ein Gefäß unter V4 gestellt und durch vorsichtiges Öffnen von V4 die Probe in das Gefäß zur weiteren Untersuchung gefüllt. Anschließend wird V4 wieder geschlossen.

Die Probe wird sowohl vor Schritt 1 als auch nach Schritt 3 zur Bestimmung des Staubanteils vor (S_{vorA}) und nach Attrition (S_{nachA}) der Bestimmung nach DIN 55992-2 unterzogen.

| | |
|---|---|
| Der "Attrition-Index" A; ergibt sich aus | Aⱼ = S_{nachA} / S_{vorA} |
| Die "Attrition-Differenz" Ad ergibt sich aus | A_{d} = S_{nachA} - S_{vorA} |

### BEISPIELE

In den nachfolgenden Beispielen wird als Vorprodukt eine teilchenförmige, schwach vernetzte, teilneutralisierte Polyacrylsäure eingesetzt, die bei der Firma Stockhausen GmbH & Co. KG unter der Handelsbezeichnung FAVOR® des Typs Z3050 kommerziell erhältlich ist. Diese besitzt eine Retention von 33 g/g und einen Staubanteil von 3,7.

### BEISPIEL 1

Herstellung der erfindungsgemäßen pulverförmigen wasserabsorbierenden Polymers:

1.000 g des Vorprodukts wurden mit den in der Tabelle 1 angegebenen Mengen an Feinteilchen und thermoplastischem Klebstoff in einem Vertikalmischer (MTI-Mischtechnik Industrieanlagen GmbH, Typ LM 1.5/5) vorgelegt und bei 750 upm mit 40 g 25 Gew.-%iger Ethylencarbonatlösung versetzt.

Das beschichtete Vorprodukt wurde in einem Gericke-Pulverförderer (Gericke GmbH, Typ GLD 75) überführt und kontinuierlich mit einer Förderleistung von 20 g/min in einen Labor-Nara-Reaktor (HTM Reetz GmbH, Typ Laborpaddelmischer "Mini-Nara II") gefördert. Die Verweilzeit im Trockner betrug ca. 90 Minuten und das Produkt wird auf 185-195°C Maximaltemperatur erhitzt. Während dieser Zeit trat die Nachvernetzung ein und der thermoplastische Klebstoff wurde geschmolzen, beeinflusste das Fließverhalten, verteilte sich auf der Oberfläche und band die Feinteilchen an der Oberfläche der wasserabsorbierenden Polymerteilchen.

**Tabelle 1**

| Versuch Nr. | Feinteilchen | Menge an Feinteilchen | Thermoplast. Klebstoff | Menge an thermoplastischem Klebstoff | FFC | Staubanteil |
|---|---|---|---|---|---|---|
| 1 (HBr 7/127) | MgHPO₄·3H₂ O (< 50 µm) | 1,0Gew.-% | SchaettiFix^{®} 386¹⁾ | 0,5 Gew.-% | 5,4 | 0,2 |
| 2 (HBr 7/128) | Technocell^{®} 75 (CelluloseFaser < 75 µm) | 1,0Gew.-% | SchaettiFix^{®} 386¹⁾ | 0,5 Gew.-% | 4,3 | 0,1 |

| | | | | | | |
|---|---|---|---|---|---|---|
| ¹⁾ Produkt der Firma Schaetti AG, Zürich, Schweiz; Schmelzbereich: 130-160°C, Schmelzviskosität bei 160°C: 520 Pa·sec, thermische Stabilität: 120°C. | | | | | | |

### BEISPIEL 2

Die in der Tabelle 2 zusammengestellten Ergebnisse wurden wie unter Beispiel 1 beschrieben mit den in Tabelle 2 angegebenen Stoffen und Mengen und 60g der 25 Gew.-%igen Ethylencarbonatlösung hergestellt. Die Proben wurden dem Attrition-Test unterzogen. Der FFC-Wert wurde nach Durchführung des Attrition-Tests bestimmt. Der CRC-Wert wurde vor und nach Durchführung des Attrition-Tests bestimmt.

**Tabelle 2**

| | Kontrolle | Vestamelt^{®2)}4481 | SchaettiFix^{®} 386 |
|---|---|---|---|
| Versuch Nr. | 3 | 4 | 5 |
| Menge an thermoplastischem Klebstoff | | 0,3 Gew.-% | 0,3 Gew.-% |
| Staubanteil³⁾ S_{vorA} | 0,44 | 0,40 | 0,39 |
| Staubanteil³⁾ S_{nachA} | 7,53 | 3,48 | 3,05 |
| Aᵢ | 17,11 | 8,7 | 8,97 |
| A_{d} | 7,09 | 3,08 | 2,66 |
| FFC vor Attrition-Test | 8,7 | 4,6 | 5,0 |
| CRC-Wert vor Attrition-Test [g/g] | 27,3 | 27,5 | 27,2 |

| | | | |
|---|---|---|---|
| ²⁾ Produkt der Degussa AG, Düsseldorf, Deutschland ³⁾ Vierfachbestimmung | | | |

### BEISPIEL 3

Herstellung eines pulverförmigen wasserabsorbierenden Polymers durch Beschichtung mit einem thermoplastischen Klebstoff in Gegenwart von Superabsorberfeinteilchen:

1.000 g des Vorprodukts wurde mit der in der Tabelle 3 angegebenen Menge an thermoplastischem Klebstoff in einem Vertikalmischer (MTI-Mischtechnik Industrieanlagen GmbH; Typ LM 1.5/5) vorgelegt und bei 750 rpm mit 40 g 25 Gew.-%iger Ethylencarbonatlösung beschichtet.

Das beschichtete Vorprodukt wurde in einem Gericke-Pulverförderer (Gericke GmbH, Typ GLD 75) überführt und kontinuierlich mit einer Förderleistung von 20 g/min in einen Labor-Nara-Reaktor (HTM Reetz GmbH, Typ Laborpaddelmischer "Mini-Nara II") gefördert. Die Verweilzeit im Trockner betrug ca. 90 Minuten und das Produkt wird auf 185-195°C Maximaltemperatur erhitzt. Während dieser Zeit trat die Nachvernetzung ein und der thermoplastische Klebstoff wurde geschmolzen, beeinflusste das Fließverhalten und verteilte sich auf der Oberfläche.

**Tabelle 3**

| Versuch Nr. | Feinteilchen | Menge an Feinteilchen | Thermoplast. Klebstoff | Menge an thermoplastischem Klebstoff | FFC | Staubanteil |
|---|---|---|---|---|---|---|
| 6 (HBr 7/126) | - | - | SchaettiFix^{®} 386 | 0,5 Gew.-% | 5,1 | 0,4 |
| 7 Kontrolle (HBr 7/125) | - | - | - | - | - | 4,5 |

Die Beispiele zeigen, dass durch die Verwendung von thermoplastischen Klebstoffen sowohl das wasserabsorbierende Polymer begleitende Superabsorberfeinteilchen als auch weitere von dem wasserabsorbierenden Polymer verschiedene Feinteilchen an wasserabsorbierende Polymerteilchen mit hoher Stabilität und Verringerung des Staubanteils gebunden werden können, ohne dass die Saugeigenschaften wie Retention des so erhaltenen wasserabsorbierenden Polymerteilchens oder Fließfähigkeit leiden.

## Patentansprüche

1. Pulverförmige wasserabsorbierende Polymere, beinhaltend als Bestandteile:
- 0,01 bis 20 Gew.-% eines Feinteilchens mit einer Teilchengröße von weniger als 200 µm,
- 0,001 bis 10 Gew.-% eines thermoplastischen Klebstoffs,
- 60 bis 99,989 Gew.-% eines wasserabsorbierenden Polymerteilchens mit einer
Teilchengröße von 200 µm und mehr,
wobei
die Feinteilchen mit der Oberfläche des wasserabsorbierenden Polymerteilchens über den thermoplastischen Schmelzklebstoff verbunden sind und die pulverförmigen wasserabsorbierenden Polymere entweder
- einen Fließ-Wert (FFC) im Bereich von 1 bis 13, oder
- einen Staubanteil von höchstens 6 zeigen.

2. Pulverförmige wasserabsorbierende Polymere nach Anspruch 1, mit einem Fließ-Wert (FFC) im Bereich von 1 bis 13 und einem Staubanteil von höchstens 6.

3. Pulverförmige wasserabsorbierende Polymere nach Anspruch 1 oder 2, wobei der thermoplastische Klebstoff eine Schmelztemperatur nach ISO 11357 von mindestens 50°C, vorzugsweise von mindestens 60°C und darüber hinaus bevorzugt von mindestens 70°C aufweist.

4. Pulverförmige wasserabsorbierende Polymere nach einem der vorhergehenden Ansprüche, wobei der thermoplastische Klebstoff eine Schmelzviskosität nach Brookfield (ASTM E 28) mit einer 27ger Spindel bei einer Temperatur von 160°C, kleiner als 2000 Pas aufweist.

5. Pulverförmige, wasserabsorbierende Polymere nach einem der vorhergehenden Ansprüche, wobei der thermoplastische Klebstoff zu mindestens 10 Gew.-%, vorzugsweise mindestens 50 Gew.-% und besonders bevorzugt mindestens 90 Gew.-% ein Polykondensat aufweist.

6. Pulverförmige, wasserabsorbierende Polymere nach Anspruch 5, wobei das Polykondensat ein Polyester ist.

7. Pulverförmige wasserabsorbierende Polymere nach einem der vorhergehenden Ansprüche, wobei das Feinteilchen zu mindestens 80 Gew.-%, bezogen auf das Gewicht des Feinteilchens, ein organisches Feinteilchen beinhaltet.

8. Pulverförmige wasserabsorbierende Polymere nach einem der vorhergehenden Ansprüche, wobei das Feinteilchen zu mindestens 80 Gew.-%, bezogen auf das Gewicht des Feinteilchens, ein anorganisches Feinteilchen beinhaltet.

9. Pulverförmige wasserabsorbierende Polymere nach einem der vorhergehenden Ansprüche, wobei diese im Oberflächenbereich durch einen Oberflächenvernetzer nachvernetzt sind.

10. Pulverförmige wasserabsorbierende Polymere nach Anspruch 9, wobei der Oberflächenvemetzer mindestens eine organische Verbindung oder mindestens ein polyvalentes Metallkation beinhaltet.

11. Verfahren zur Herstellung pulverförmiger wasserabsorbierender Polymere, wobei als Bestandteile:
- 0,01 bis 20 Gew.-% eines Feinteilchens mit einer Teilchengröße von weniger als 200 um,
- 0,001 bis 10 Gew.-% eines thermoplastischen Klebstoffs,
- 60 bis 99,989 Gew.-% eines wasserabsorbierenden Polymerteilchens mit einer
Teilchengröße von 200 µm und mehr,
bei einer Temperatur im Bereich von 120 bis 250 °C, bevorzugt 150 bis 220 °C und besonders bevorzugt 170 bis 200 °C miteinander in Kontakt gebracht werden.

12. Verfahren nach Anspruch 11, wobei das in Kontakt bringen in einem kontinuierlich fördernden Mischofen erfolgt.

13. Verfahren nach Anspruch 12, wobei der Ofen bewegliche, beheizte Fördermittel aufweist.

14. Verfahren nach einem der Ansprüche 11 bis 13, wobei als weiterer Bestandteil ein Nachvernetzer in Kontakt gebracht wird.

15. Verfahren nach Anspruch 14, wobei der Nachvernetzer und der thermoplastische Klebstoff gemeinsam den anderen Bestandteilen zugeführt werden.

16. Verfahren nach Anspruch 14 oder 15, wobei mindestens der Nachvernetzer in einer flüssigen Phase vorliegt.

17. Verfahren zur Herstellung eines pulverförmiges wasserabsorbierendes Polymer nach Anspruch 11, wobei dieses Polymer vorzugsweise **gekennzeichnet ist durch**:
P1 einen Fließ-Wert (FFC) im Bereich von 1 bis 13; oder
P2 einen Staubanteil von höchstens 6,
P3 einem Attrition-Index Aᵢ im Bereich von 1 bis 17,
P4 einer Attrition-Differenz A_{d} im Bereich von 0 bis 7,
P5 einer Retention bestimmt nach ERT 441.1 -99 von mindestens 20 g/g.

## Claims

1. Powdery, water-absorbing polymers comprising as constituents:
- 0.01 to 20% by weight of a fine particle having a particle size of less than 200 µm,
- 0.001 to 10% by weight of a thermoplastic adhesive,
- 60 to 99.989% by weight of a water-absorbing polymer particle having a particle size of 200 µm and greater,
wherein
the fine particles are bound to the surface of the water-absorbing polymer particle by the thermoplastic melt adhesive and the powdery waterabsorbing polymers show either
- a flow coefficient (FFC) in the range of 1 to 13, or
- a dust content of at most 6.

2. Powdery, water-absorbing polymers according to Claim 1, having a flow coefficient (FFC) in the range of 1 to 13 and a dust content of at most 6.

3. Powdery, water-absorbing polymers according to Claim 1 or 2, wherein the thermoplastic adhesive has a melting temperature according to ISO 11357 of at least 50°C, preferably at least 60°C and more preferably at least 70°C.

4. Powdery, water-absorbing polymers according to any of the preceding claims, wherein the thermoplastic adhesive has a Brookfield melt viscosity (ASTM E 28) using spindle number 27 of less than 2000 Pas at a temperature of 160°C.

5. Powdery, water-absorbing polymers according to any of the preceding claims, wherein the thermoplastic adhesive has at least 10% by weight, preferably at least 50% by weight and particularly preferably at least 90% by weight of a polycondensate.

6. Powdery, water-absorbing polymers according to Claim 5, wherein the polycondensate is a polyester.

7. Powdery, water-absorbing polymers according to any of the preceding claims, wherein the fine particle comprises at least 80% by weight of an organic fine particle, based on the weight of the fine particle.

8. Powdery, water-absorbing polymers according to any of the preceding claims, wherein the fine particle comprises at least 80% by weight of an inorganic fine particle, based on the weight of the fine particle.

9. Powdery, water-absorbing polymers according to any of the preceding claims, wherein said polymers are cross-linked in the surface region by a surface cross-linker.

10. Powdery, water-absorbing polymers according to Claim 9, wherein the surface cross-linker comprises at least one organic compound or at least one polyvalent metal cation.

11. Method for preparing powdery, water-absorbing polymers, wherein the constituents:
- 0.01 to 20% by weight of a fine particle having a particle size of less than 200 µm,
- 0.001 to 10% by weight of a thermoplastic adhesive,
- 60 to 99.989% by weight of a water-absorbing polymer particle having a particle size of 200 µm and greater,
are brought into contact with one another at a temperature in the range of 120 to 250°C, preferably 150 to 220°C and particularly preferably 170 to 200°C.

12. Method according to Claim 11, wherein said constituents are brought into contact in a continuously conveying mixing oven.

13. Method according to Claim 12, wherein the oven has mobile, heated conveying means.

14. Method according to any of Claims 11 to 13, wherein a cross-linker is brought into contact as a further constituent.

15. Method according to Claim 14, wherein the crosslinker and the thermoplastic adhesive are fed together with the other constituents.

16. Method according to Claim 14 or 15, wherein at least the cross-linker is present in a liquid phase.

17. Method for preparing a powdery, water-absorbing polymer according to Claim 11, wherein this polymer is preferably **characterized by**:
P1 a flow coefficient (FFC) in the range of 1 to 13; or
P2 a dust content of at most 6,
P3 an attrition index Aᵢ in the range of 1 to 17,
P4 an attrition difference A_{d} in the range of 0 to 7,
P5 a retention of at least 20 g/g determined by ERT 441.1 -99.

## Revendications

1. Polymères pulvérulents absorbant l'eau, comportant comme composants :
- 0,01 à 20 % en poids d'une fine particule ayant une taille de particule de moins de 200 µm,
- 0,001 à 10 % en poids d'un adhésif thermoplastique,
- 60 à 99,989 % en poids d'une particule de polymère absorbant l'eau, ayant une taille de particule de 200 µm et plus,
les fines particules étant liées à la surface de la particule de polymère absorbant l'eau par l'adhésif fusible thermoplastique et les polymères pulvérulents absorbant l'eau présentant soit
- un indice d'écoulement (FFC) dans la plage de 1 à 13, soit
- une proportion de poussière d'au maximum 6.

2. Polymères pulvérulents absorbant l'eau selon la revendication 1, ayant un indice d'écoulement (FFC) dans la plage de 1 à 13 et une proportion de poussière d'au maximum 6.

3. Polymères pulvérulents absorbant l'eau selon la revendication 1 ou 2, dans lesquels l'adhésif thermoplastique présente une température de fusion selon ISO 11357 d'au moins 50 °C, de préférence d'au moins 60 °C et encore mieux d'au moins 70 °C.

4. Polymères pulvérulents absorbant l'eau selon l'une quelconque des revendications précédentes, dans lesquels l'adhésif thermoplastique présente une viscosité à chaud selon Brookfield (ASTM E 28) avec une broche 27, à une température de 160 °C, inférieure à 2 000 Pa.s.

5. Polymères pulvérulents absorbant l'eau selon l'une quelconque des revendications précédentes, dans lesquels l'adhésif thermoplastique comporte à raison d'au moins 10 % en poids, de préférence d'au moins 50 % en poids et de façon particulièrement préférée d'au moins 90 % en poids un produit de polycondensation.

6. Polymères pulvérulents absorbant l'eau selon la revendication 5, dans lesquels le produit de polycondensation est un polyester.

7. Polymères pulvérulents absorbant l'eau selon l'une quelconque des revendications précédentes, dans lesquels la fine particule comprend à raison d'au moins 80 % en poids, par rapport au poids de la fine particule, une fine particule organique.

8. Polymères pulvérulents absorbant l'eau selon l'une quelconque des revendications précédentes, dans lesquels la fine particule comprend à raison d'au moins 80 % en poids, par rapport au poids de la fine particule, une fine particule inorganique.

9. Polymères pulvérulents absorbant l'eau selon l'une quelconque des revendications précédentes, ceux-ci étant post-réticulés dans la zone superficielle par un agent de réticulation superficielle.

10. Polymères pulvérulents absorbant l'eau selon la revendication 9, dans lesquels l'agent de réticulation superficielle comprend au moins un composé organique ou au moins un cation métallique plurivalent.

11. Procédé pour la production de polymères pulvérulents absorbant l'eau, dans lequel on met en contact entre eux en tant que composants :
- 0,01 à 20 % en poids d'une fine particule ayant une taille de particule de moins de 200 µm,
- 0,001 à 10 % en poids d'un adhésif thermoplastique,
- 60 à 99,989 % en poids d'une particule de polymère absorbant l'eau, ayant une taille de particule de 200 µm et plus,
à une température dans la plage de 120 à 250 °C, de préférence de 150 à 220 °C et de façon particulièrement préférée de 170 à 200 °C.

12. Procédé selon la revendication 1, dans lequel la mise en contact s'effectue dans un four mixte à acheminement continu.

13. Procédé selon la revendication 12, dans lequel le four comporte des moyens d'acheminement mobiles, chauffés.

14. Procédé selon l'une quelconque des revendications 11 à 13, dans lequel en tant qu'autre composant un agent de post-réticulation est mis en contact.

15. Procédé selon la revendication 14, dans lequel l'agent de post-réticulation et l'adhésif thermoplastique sont ajoutés ensemble aux autres composants.

16. Procédé selon la revendication 14 ou 15, dans lequel au moins l'agent de post-réticulation se trouve en une phase liquide.

17. Procédé pour la production d'un polymère pulvérulent absorbant l'eau selon la revendication 11, ce polymère étant de préférence **caractérisé par** :
P1 un indice d'écoulement (FFC) dans la plage de 1 à 13 ; ou
P2 une proportion de poussière d'au maximum 6,
P3 un indice d'attrition Aᵢ dans la plage de 1 à 17,
P4 une différence d'attrition A_{d} dans la plage de 0 à 7,
P5 une rétention, déterminée selon ERT 441.-99, d'au moins 20 g/g.
